# EUROPEAN PATENT APPLICATION

(11) **EP 4 821 424 A1**
(43) Date of publication of application: **14.10.2026**
(21) Application number: 25290014.7
(22) Date of filing: 07.04.2025
(51) Int. Cl.: A61B 3/00, A61B 3/10, A61B 3/103, A61B 3/107

(54) **NEW DECOMPOSITION BASE FOR A METHOD AND DEVICE FOR ANALYSING THE SHAPE OF AN OBJECT**

(71) Applicant: Gatinel, Damien, 75006 Paris (FR); Malet, Jacques, 75014 Paris (FR)
(72) Inventor: Gatinel, Damien, 75006 Paris (FR); Malet, Jacques, 75014 Paris (FR)
(74) Representative: Calysta NV

(57) **Abstract**

Method comprising the following steps: providing a derivative wavefront map of an object (S1); computing a set of coefficients representing the derivative wavefront map (S2), wherein each coefficient (gj) is related to a base function (Gj) of a set of base functions so that the linear combination of the set of base functions (Gj) each weighted by the coefficient (gj) of its base function (Gj) represents the derivative wavefront map (V); and outputting the set of coefficients (S3).

## Description

### Technical Field

The present invention relates to a method, computer program and device for analyzing the shape of an object by decomposing a map of the object in a set of base functions. The present invention is applied preferably for lens objects, preferably for ophthalmic lenses, preferably for eye lenses.

### Prior art

Zernike polynomials are well known to decompose wavefront maps of lens objects.

In order to improve the application of Zernike polynomials for ophthalmology, the article "Polynomial decomposition method for ocular wavefront analysis" from Gatinel et al published in the Journal of Optical Society of America A, Vol. 35, No. 12 / December 2018, page 2035 proposes remove low degree (LD) components from higher degree Zernike functions on the cost of losing the orthogonality of the Zernike functions in order to improve the clinical interpretability of the Zernike coefficients.

Radial vergence maps offer a powerful, clinician-friendly approach to analyzing and correcting ocular aberrations. By providing a direct, dioptric representation of wavefront data, they align closely with subjective refraction, enable systematic decomposition with orthogonal functions, and remain robust across aberration types-all while simplifying the derivation of refractive prescriptions. These benefits make radial vergence maps an efficient, intuitive, and accurate tool, surpassing traditional wavefront methods in practical utility and enhancing vision care outcomes. However, Zernike polynomials cannot be applied to radial vergence maps due to the base functions describing tilt which generate problems for the vergence map depending on the inverse radius. This is due to the division by r=0 that occurs for the tilt. The derivation of the tilt leads to a radial constant which one cannot divide by r when r is 0.

Two publications have described methods for decomposing vergence maps into elementary functions.

The article "Describing ocular aberrations with wavefront vergence maps" of Nam et al published in clinical and experimental optometry 2009, 92, 3 pages 194-205 uses a basis directly derived from Zernike polynomials: it includes terms of degree 1 (tilt) and generates thus the same problems as the Zernike polynomials for vergence maps.

The article "Objective refraction from monochromatic wavefront aberrations via Zernike power polynomials" published in Ophthal. Physiol. Opt. 2007, issue 27, pages 245-255 apply the derivative formulas used for calculating vergence (and those used for calculating curvature expressed in keratometric diopters) to Zernike modes. However, the resulting base functions lack the mathematical properties of the Zernike polynomials which make their corresponding coefficients easily interpretable and easily computable.

### Brief summary of the invention

It is the object of the invention to provide an improved decomposition basis for derivative wavefront maps like vergence maps or curvature maps.

The object is solved by a method, a computer program and a device according to the independent claims.

In contrast to Zernike base functions, the base functions according to the invention have been formulated to describe derivative wavefront maps which are clinically more easily interpretable. These adapted base functions have been significantly improved compared to the ones in the state of the art so that they can be used easily by practitioners which are not always mathematicians, for example by clinicians for analyzing ophthalmic lenses and eyes. The improvement involves either the orthogonalization of the high order base functions or the normalization of the base functions or both. The orthogonalization of the high degree (HD) base functions makes the calculation of the HD coefficients very easy using the scalar product. In addition, it improves the interpretability of the HD coefficients as a variation of one HD coefficient does not influence the others. Also the normalization significantly improves the interpretability of the coefficients for practitioners like clinicians as the coefficients become better comparable. In the example of clinicians, they could use the value of coefficients right away as the value of the power of lens prescriptions or surgery for a certain lens aberration/correction.

The object is solved further by a method comprising the following steps: Providing a derivative wavefront map of an object; computing a set of coefficients representing the derivative wavefront map, wherein each coefficient is related to a base function of a set of base functions so that the linear combination of the set of base functions each weighted by the coefficient of its base function represents the derivative wavefront map, and outputting the set of coefficients, wherein the set of base functions comprises base functions *Gⱼ*(*r*, *t*) with j=1, ..., J depending on the radius r and the angle t, wherein the method comprises one or more of the following embodiments.

The object is solved further by a computer program comprising instructions configured to perform the following steps, when executed on a processor: providing a derivative wavefront map of an object; computing a set of coefficients representing the derivative wavefront map, wherein each coefficient is related to a base function of a set of base functions so that the linear combination of the set of base functions each weighted by the coefficient of its base function represents the derivative wavefront map, and outputting the set of coefficients, wherein the set of base functions comprises base functions *Gⱼ*(*r*,*t*) with j=1, ..., J depending on the radius r and the angle t, wherein the computer program performs one or more steps of the following embodiments.

The object is solved further by a device or system comprising: a map provider for providing a derivative wavefront map of an object; a coefficient computer for computing a set of coefficients representing the derivative wavefront map, wherein each coefficient is related to a base function of a set of base functions so that the linear combination of the set of base functions each weighted by the coefficient of its base function represents the derivative wavefront map; and an output section for outputting the set of coefficients; wherein the set of base functions comprises base functions *Gⱼ*(*r*, *t*) with j=1, ..., J depending on the radius r and the angle t, wherein the method comprises one or more of the following embodiments.

The following embodiment refers to further features of the above method, computer program and system or device.

In one embodiment, the set of base functions comprises low degree base functions and high degree base functions.

In one embodiment, the j-th high degree coefficients gj(V) associated to the j-th high degree base functions Gj(r, t) are computed based on the scalar product of the j-th high degree base function Gj(r, t) with the derivative wavefront map or with a residual high degree derivative wavefront map.

In one embodiment, the set of base functions constitutes an orthogonal basis, or formulated differently, each high degree base function is orthogonal to each low degree base function. A full orthogonal set of base functions makes the computation of the coefficients easier by using the scalar product. In addition, the base functions describe more precisely the derivative wavefront map. This decomposition guarantees a mathematically clean separation between all base functions and thus between all obtained coefficients. This full orthogonal set of base functions might be advantageous in some cases.

In one embodiment, at last some of the high degree base functions are not orthogonal to the low degree base functions, and/or the high degree base functions have no components independent of the radius r or have no components of order zero with respect to the radius, and/or each component of each high degree base function depends on the radius r, and/or . Having a fully orthogonal set of base functions has also some disadvantages as the orthogonality generates in HD base functions components of degree zero, i.e. components of defocus and astigmatism. Clinically, it can thus be advantageous to have rather a non-orthogonal set of base functions which does not mix any LD components in the HD base functions. The LD coefficients represent thus the full and only components of defocus and astigmatism. Since they are clinically the most relevant, it is thus advantageous to represent those each with one coefficient. The remaining HD base functions are strictly orthogonal between themselves treating the remaining residual derivative wavefront map with an orthogonal set of HD base functions.

In one embodiment, the low degree base functions are orthogonal to all other low degree base functions. This holds preferably for both embodiments, first for the embodiment with a full set of orthogonal base functions and second, for the embodiment with at least some high degree base functions being non-orthogonal to the low degree base functions.

In one embodiment, the step of computing the coefficients comprises the steps of: Estimating the coefficients of the low degree base functions for the derivative map as low degree coefficients; computing the low degree derivative wavefront map based on the low degree coefficients and the low degree base functions; determining a high degree derivative wavefront map based on the derivative wavefront map and the low degree derivative wavefront map, preferably by subtracting the estimated low degree derivative wavefront map from the derivative wavefront map; computing the coefficients of the high degree base functions for the high degree derivative wavefront map as high degree coefficients. This approach is very robust and clinically better interpretable than a fully orthogonal base. The set of low degree coefficients is obtained by an estimation, preferably a fitting. The remaining high degree coefficients are obtained efficiently by an orthogonal set of base functions based on the high degree derivative wavefront map describing the high degree derivative wavefront map mathematically precise with an orthogonal set of base functions without LD components of defocus and astigmatism.

In one embodiment, the low degree coefficients are estimated by fitting the linear combination of the three low degree base functions weighted by the three low degree coefficients (short fitting of the LD base functions) to the derivative wavefront maps. In a preferred embodiment, a central portion/zone of the derivative wavefront map is considered more relevant than an external portion/zone of the derivative wavefront map for this LD fitting. In the simplest case, the LD base functions are fitted only to the central portion of the derivative wavefront map (without considering the external portion for the fitting at all). However, it could also be that the external portion/zone is simply weighted less for the fitting than the central portion. The central portion is preferably a zone of the derivative wavefront map within a central portion radius. The central portion radius is smaller than the maximum radius of the derivative wavefront map, preferably smaller than 50% of the maximum radius, preferably smaller than 40% of the maximum radius, preferably smaller than 30% of the maximum radius, preferably smaller than 25% of the maximum radius. The external portion is the portion of the derivative wavefront map with a radius larger than the central portion radius. In this embodiment, a localized fit with the orthogonal low degree base functions is applied (only) within the central region (or at least with a stronger weight in the central region). This ensures that the osculating component accurately describes the summit curvature or vergence without being influenced by more peripheral variations. This is particular interesting for applications in ophthalmics as the defocus and astigmatism are defined mainly by the shape of the central region. Thus, a correction lens selected based on the low degree coefficients fitted mainly on the central portion provides the best value for this correction lens. The obtained LD components obtained from this fitting are then used to obtain a HD derivative wavefront map in order to then estimate the HD coefficient precisely with an orthogonal set of HD base functions. These higher-order functions are strictly orthogonal between themselves, ensuring that no interference remains from the previously removed low-degree component. This decomposition guarantees a mathematically clean separation between the clinically relevant low-degree fit and the higher-order contributions, which describe localized aberrations or surface irregularities. The low-degree fit provides a robust representation of the corneal or ocular apex curvature/refraction, aligning with clinically meaningful interpretations of central vergence and curvature. This structured separation of low- and high-degree components allows for a clear, hierarchical description of optical properties, ensuring both clinical relevance and mathematical rigor in the analysis of vergence and curvature distributions. This method effectively combines the advantages of a localized, apex-centered fit with a globally orthogonal decomposition for the remaining higher-order terms, ensuring a clinically meaningful and numerically robust analysis.

In one embodiment, a peripheral component/zone of the derivative wavefront map is fit to the HD coefficients and then subtracted to the total wavefront derivative map. This remainder is then fitted to the LD coefficients.

In one embodiment, the set of high degree base functions is orthogonal to each other. The orthogonalization of the high degree (HD) base functions makes the calculation of the HD coefficients very easy using the scalar product. In addition, it improves the interpretability of the HD coefficients as a variation of one HD coefficient does not influence the others. This decomposition guarantees a mathematically clean separation between the high degree contributions, which describe localized aberrations or surface irregularities.

In one embodiment, the set of base functions are normalized. Also, the normalization significantly improves the interpretability of the coefficients for practitioners like clinicians as the coefficients become better comparable. In the example of clinicians, they could use the value of coefficients right away as the value of the power of lens prescriptions or surgery for a certain lens aberration/correction.

In one embodiment, the coefficients are output in dioptre. This allows clinicians to use the coefficients directly to select respective correction lenses for the respective aberrations related to the base functions with high dioptre values, especially if the base functions are normalized.

In one embodiment, the set of base functions comprise low degree base functions, wherein the low degree base functions comprise *G₁*(*r*, *t*) = *a*₁ sin(2*t*) with *a*₁ ≠ 0, *G₂*(*r*, *t*) = *a*₂ with *a*₂ ≠ 0 and *G₃*(*r*, *t*) = *a*₃ cos(2*t*) with *a*₃ ≠ 0. These functions describe defocus (j=2) and oblique and vertical astigmatism (j=1 and j=3). The LD coefficients gj j=1, ..., 3 describe thus the strength of these three aberrations. Preferably, the multiplicator *a*₁ = *a*₃. Preferably, the multiplicator ${a}_{3} = \sqrt{2}$ and/or ${a}_{1} = \sqrt{2}$. Preferably, the multiplicator a₂ = 1. This allows to provide a set of normalized base functions.

In one embodiment, the high degree base functions comprise *G₄*(*r*, *t*) = *b*₄*r* sin(3*t*) with *b*₄ ≠ 0, *G₅*(*r*, *t*) = *b*₅*r* sin(*t*) with *b*₅ ≠ 0, *G₆*(*r*, *t*) = *b*₆*r* cos(*t*) with *b*₆ ≠ 0 and *G₇*(*r*, *t*) = *b*₇*r* cos(3*t*) with *b*₇ ≠ 0. Preferably, the multiplicators *bⱼ =* 2 for j= 4, ..., 7. This allows to provide a set of normalized base functions.

In one embodiment, the high degree base functions comprise *G₈*(*r*, *t*) = *c*₈*r*² sin(4*t*) with *c*₈ ≠ 0, *G₉*(*r*, *t*) *=* (*c*₉*r*² + *a*₉) sin(2*t*) with c₉ ≠ 0, ${a}_{9} ∈ ℝ$, *G₁₀*(*r*, *t*) *=* (*c*₁₀*r*² + *a*₁₀) with *c*₁₀ *≠* 0 ${a}_{10} ∈ ℝ$, *G*₁₁(*r*,*t*) *=* (*c*₁₁*r*² + *a*₁₁) cos(2*t*) with *c*₁₁ ≠ 0, ${a}_{11} ∈ ℝ$ and *G₁₂*(*r, t*) *= c*₁₂*r*² cos(4*t*) with *c*₁₂ ≠ 0. Preferably for the multiplicators hold one or more of the following constraints/conditions: the multiplicators *c*₈ = *c*₁₂ and/or *c*₉ = *c*₁₁ and/or ${c}_{8} = \sqrt{6}$ and/or ${c}_{12} = \sqrt{6}$.

In one embodiment, the multiplicators a*ⱼ* ≠ 0 for j= 9, ..., 11 . Preferably for the multiplicators hold one or more or all of the following constraints/conditions: ${c}_{9} = 2 \sqrt{6}$ and/or ${c}_{11} = 2 \sqrt{6}$ and/or ${c}_{10} = 2 \sqrt{3}$ and/or ${a}_{9} = {a}_{11} = - \sqrt{6}$ and/or *a*₁₀ = $- \sqrt{3}$. This allows to provide a fully orthogonal set of normalized base functions.

In one embodiment, the multiplicators are a*ⱼ =* 0 for j= 9, ..., 11 . This might destroy the orthogonality between the base functions Gj for j=9, ... , 11, but removes LD components in the HD base functions and improves thus the interpretability of the LD coefficients obtained. Preferably, for the multiplicators hold one or more or all of the following constraints/conditions: ${c}_{9} = \sqrt{6}$ and/or ${c}_{11} = \sqrt{6}$ and/or ${c}_{10} = \sqrt{3}$ .

In one embodiment, the high degree base functions comprise the base functions ${G}_{13} \left(r, t\right) = {d}_{13} {r}^{3} sin \left(5t\right) with {d}_{13} \neq 0 ,$ ${G}_{14} \left(r, t\right) = \left({d}_{14}{r}^{3}-{b}_{14}r\right) sin \left(3t\right) with {d}_{14} \neq 0 , {b}_{14} \neq 0 ,$ ${G}_{15} \left(r, t\right) = \left({d}_{15}{r}^{3}-{b}_{15}r\right) sin \left(t\right) with {d}_{15} \neq 0 , {b}_{15} \neq 0 ,$ ${G}_{16} \left(r, t\right) = \left({d}_{16}{r}^{3}-{b}_{16}r\right) cos \left(t\right) with {d}_{16} \neq 0 , {b}_{16} \neq 0 ,$ ${G}_{17} \left(r, t\right) = \left({d}_{17}{r}^{3}-{b}_{17}r\right) cos \left(3t\right) with {d}_{17} \neq 0 , {b}_{17} \neq 0 and$ ${G}_{18} \left(r, t\right) = {d}_{18} {r}^{3} cos \left(5t\right) with {d}_{18} \neq 0 .$

In one embodiment, the multiplicators are ${d}_{18} = {d}_{13} = 2 \sqrt{2}$, *dⱼ =* $6 \sqrt{2}$ for *j* = 14,..,17, and ${b}_{j} = - 4 \sqrt{2}$ for*j* = 14,..,17.

In one embodiment, the high degree base functions comprise the base functions ${G}_{19} \left(r, t\right) = {e}_{19} {r}^{4} sin \left(6t\right) with {e}_{19} \neq 0 ,$ ${G}_{20} \left(r, t\right) = \left({e}_{20}{r}^{4}+{c}_{20}{r}^{2}\right) sin \left(4t\right) with {e}_{20} , {c}_{20} \neq 0 ,$ ${G}_{21} \left(r, t\right) = \left({e}_{21}{r}^{4}+{c}_{21}{r}^{2}+{a}_{21}\right) sin \left(2t\right) with {e}_{21} , {c}_{21} \neq 0 , {a}_{21} ∈ ℝ ,$ ${G}_{22} \left(r, t\right) = \left({e}_{22}{r}^{4}+{c}_{22}{r}^{2}+{a}_{22}\right) with {e}_{22} , {c}_{22} \neq 0 , {a}_{22} ∈ ℝ ,$ ${G}_{23} \left(r, t\right) = \left({e}_{23}{r}^{4}+{c}_{23}{r}^{2}+{a}_{23}\right) cos \left(2t\right) with {e}_{23} , {c}_{23} \neq 0 , {a}_{23} ∈ ℝ ,$ ${G}_{24} \left(r, t\right) = \left({e}_{24}{r}^{4}+{c}_{24}{r}^{2}\right) cos \left(4t\right) with {e}_{24} , {c}_{24} \neq 0 and$ ${G}_{25} \left(r, t\right) = {e}_{25} {r}^{4} cos \left(6t\right) with {e}_{25} \neq 0 .$

In one embodiment, the multiplicators fulfill one or more of the following conditions: ${e}_{19} = {e}_{25} = \sqrt{10}$ and/or ${e}_{20} = {e}_{24} = 4 \sqrt{10}$ and/or *c*₂₀ = *c*₂₄ = $- 3 \sqrt{10}$ .

In one embodiment, the multiplicators are a*ⱼ* ≠ 0 for j= 21, ..., 23 . Preferably, multiplicators fulfill one or more of the following conditions *a*₂₁ = *a*₂₃ = $\sqrt{10}$ and ${a}_{22} = \sqrt{5}$. This allows to provide a fully orthogonal set of normalized base functions. Preferably, the multiplicators fulfill one or more of the following conditions: and/or ${e}_{21} = {e}_{23} = 6 \sqrt{10}$ and/or ${e}_{22} = 6 \sqrt{5}$ and/or ${c}_{21} = {c}_{23} = - 6 \sqrt{10}$ and/or *c*₂₂ = $- 6 \sqrt{5}$.

In one embodiment, the multiplicators are a*ⱼ* = 0 for j= 21, ...,23 . This allows to remove any LD components from the HD base functions. Preferably, the multiplicators fulfill one or more of the following conditions: ${e}_{21} = {e}_{23} = 4 \sqrt{10}$ and/or ${e}_{22} = 4 \sqrt{5}$ and/or ${c}_{21} = {c}_{23} = - 3 \sqrt{10}$ and/or ${c}_{22} = - 3 \sqrt{5}$.

In one embodiment, the device or system comprises further a measurement instrument, preferably an aberrometer, configured to measure a measurement map of the object, wherein the map provider is configured to provide the derivative wavefront map of the object based on the measurement map from the measurement instrument.

In one embodiment, the device or system comprises further a user output interface configured to output the coefficient data received from the output section. The user output interface is preferably a device for visual data output like a display. However, the user output interface could also be a communication interface to give out the output data based on the coefficients computed in a specific hardware/software protocol.

Other embodiments according to the present invention are mentioned in the appended claims and the subsequent description of an embodiment of the invention.

### Brief description of the Drawings

Fig. 1 shows a set of base functions according to a first embodiment plotted each as a circular map.
Fig. 2 shows a set of base functions according to a second embodiment plotted each as a circular map.
Fig. 3 shows a grey scale representing the amplitude of the circular maps in Fig. 1 and 2 in dioptre.
Fig. 4 shows a diagram of the steps of the method for analyzing a shape of an object according to the invention.
Fig. 5 shows a first embodiment of the step of providing the derivative wavefront map.
Fig. 6 shows a second embodiment of the step of providing the derivative wavefront map.
Fig. 7 shows a first embodiment of the step of computing the coefficients.
Fig. 8 shows an exemplary embodiment of a device for analyzing a shape of an object according to the invention.

In the drawings, the same reference numbers have been allocated to the same or analogue element.

### Detailed description of an embodiment of the invention

Other characteristics and advantages of the present invention will be derived from the non-limitative following description, and by making reference to the drawings and the examples.

The present invention has the purpose of analyzing an object. The object is preferably a curved object, preferably a lens object. Preferably, the lens object is an ophthalmic lens object. The ophthalmic lens object could be a natural or artificial ophthalmic lens object. A natural ophthalmic lens object is preferably an eye lens. The eye lens is preferably a cornea (lens) or the lens of the eye (without the cornea) or the combination of the both. The eye lens could also be the complete visual system of the eye. An artificial ophthalmic lens object could also be ophthalmic lens for improving vision like a spectacle lens, a contact lens or an intraocular lens. It would also be possible to apply the invention to other lens objects or other objects. The lens object can be a transmissive lens or a reflective lens. The lens object has preferably an optical axis which is perpendicular to the lens surface.

Curved objects are normally analyzed based on a measurement map which represents the form of the object perpendicular to an axis. In the case of a lens object, the measurement map represents the form of the lens object perpendicular to the optical axis. Such maps are thus bivariate functions, i.e. their values are described based on two variables. The map W can be represented in a cartesian coordinate system based on the coordinates x and y or in a polar coordinate system based on the coordinate radius r and angle t (equation 1) $W \left(x, y\right) = W \left(r cos\left(t\right),r sin\left(t\right)\right) = {W}_{p} \left(r, t\right)$

Subsequently, all maps are represented in the polar coordinate system without indicating this with the index p. It is clear that each of the described maps can at any time be converted from a polar coordinate system in a cartesian coordinate system or vice versa without any limitation of the invention. Subsequently, different measurement maps for such objects will be presented.

A wavefront map W(r,t) measures how light propagates through the object, preferably the lens object. The wavefront map W(r,t) is normally a wavefront error map W(r,t) which shows wavefront aberrations, i.e. deviations from the optimal lens object. The amplitude values of the wavefront maps are normally given in micrometers (short microns). The wavefront map can be measured by measurement instruments like a wavefront aberrometer, e.g. a Shack-Hartmann wavefront sensor, ray tracing aberrometer, a Tscherning aberrometer or others. wavefront maps are extensively used in ophthalmology, yet difficult to interpret by clinicians.

A derivative wavefront map V(r,t) is any map which depends on (or is a function of) first, second and/or higher derivatives of the wavefront (error) map W(r,t) of the object with respect to the radius r. (equation 2) $V \left(r, t\right) = f \left(\frac{\partial W \left(r, t\right)}{\partial r},\frac{{\partial }^{2} W \left(r, t\right)}{\partial {r}^{2}},…,\frac{{\partial }^{k} W \left(r, t\right)}{\partial {r}^{k}}\right)$ with k being the order of derivation. The derivative wavefront (error) map V(r,t) is expressed preferably in polar coordinates with a radius r and an angle t as explained above. The derivative wavefront (error) map is/are preferably circular map(s). The derivative wavefront map is preferably scaled to a unity circle, i.e. the derivative wavefront map has the maximum radius one. Analysing a derivative wavefront map has thus the advantage that tilt information is removed from the wavefront as tilt is not relevant for analysing an object like a lens. In one embodiment, the derivative wavefront map depends on the first, second or higher derivative of the wavefront (error) divided by the radius r. (equation 3) $V \left(r, t\right) = f \left(\frac{1}{r}\frac{\partial W \left(r, t\right)}{\partial r}\right)$

This removes not only the piston, but also the tilt from the wavefront. Also, the tilt is normally not interesting for analysing curved objects like lenses as it indicates only an alignment error of the measurement instrument with respect to the optical axis. In a first embodiment, the derivative wavefront map is the vergence map of the object (equation 4) $V \left(r, t\right) = \frac{i}{r} \frac{\partial W \left(r, t\right)}{\partial r}$ with i being the index of refraction of the material of the object. Since the refractive index i is a constant, it could be set to 1. However, preferably the real refractive index i of the material of the object is used which improves the interpretability of the results. A vergence map in the sense of the invention shall also cover vergence maps in which the refractive index i is set to another value than the refractive index of the actual material, e.g. i=1. Obviously, a vergence map with the correct refractive index is preferred. A vergence map in the sense of the invention shall also cover vergence maps in which the refractive index i is set to another value than the refractive index of the actual material, e.g. i=1. Obviously, a vergence map with the correct refractive index is preferred. In a second embodiment, the derivative wavefront map is the curvature map of the object (equation 5) $V \left(r, t\right) = \frac{{\partial }^{2} W \left(r, t\right)}{\partial {r}^{2}}$ which corresponds to the second derivative/differentiation of the wavefront map W(r,t) with respect to the radius r. The derivative wavefront map can be computed from a measured wavefront map or can be measured directly. For example, certain instruments measure directly the vergence map (e.g. wavefront aberrometer, autorefractor, Schreiner Principle based instruments or others) or the curvature map (e.g. topographers like corneal topographer, Scheimpflug topographer, Placido based topographer, Optical coherence topographer or others). The derivative wavefront map used in the invention can however also be calculated from another derivative wavefront map measured. For example, the vergence map can be computed from a curvature map or vice versa). When the derivative wavefront map, especially the vergence map is computed from a wavefront map, the constant offset (piston) and the tilt must be removed from the wavefront map before computing the derivative wavefront map.

It is common to decompose a wavefront map W by a weighted sum of Zernike functions (equation 6) $W = {\sum }_{n \geq 0} \left({\sum }_{m ∈ {I}_{u}} {z}_{n}^{m} \left(W\right) {Z}_{n}^{m}\right)$ with ${I}_{n} = \left\{-n,-n+2,…,+n-2,+n\right\} .$

Each Zernike function Z^{m}ₙ being a bivariate function Z^{m}ₙ (r,t), i.e. a map and each coefficient z^{m}ₙ (W) being the weight representing the related Zernike function Z^{m}ₙ in the wavefront map W. If the sum n goes from 0 to infinity, the wavefront W can be described exactly. If the sum over n goes up to a maximum index N, the Zernike representation or expansion is an approximation. The coefficients represent certain types of irregularities of the object or lens like piston (z⁰₀), tilt in y and x direction (z⁻¹₁ , z¹₁), defocus (z⁰₂), oblique astigmastism (z⁻²₂), vertical astigmatism (z²₂), trefoil and coma (n=3).

In a similar way, the present invention decomposes or represents the derivative wavefront map V(r,t) with a new set of base function (equation 7) $V \left(r, t\right) = {\sum }_{n = 2}^{N} {\sum }_{m ∈ {I}_{n}} {v}_{n}^{m} {V}_{n}^{m} \left(r, t\right)$

With the sum over the vector Iₙ defined above containing the natural numbers from -n to +n in steps of two. N is the limit of approximation, i.e. the maximum number of the index n. Each base function V^{m}ₙ being a bivariate function V^{m}ₙ (r,t), i.e. a map and each coefficient v^{m}ₙ being the weight representing the related/associated base function Z^{m}ₙ (the one with the same pair of indices m and n) in the derivative wavefront map V(r,t). The double sum could be rewritten as one sum over the index j (equation 8) $V \left(r, t\right) = {\sum }_{j = 1}^{J} {g}_{j} {G}_{j} \left(r, t\right)$ wherein each combination of n and m with n=(2, 3, 4, ..., N) and with m=(-n, -n+2, ..., n-2, n) corresponds to one index j =(1, ..., J) (see table later for N=6 and J=25). Each base function Gⱼ being a bivariate function G^{m}ₙ (r,t), i.e. a map and each coefficient gⱼ being the weight representing the related/associated base function Gⱼ (with the same index j) in the derivative wavefront map V(r,t). Thus, each coefficient gⱼ is associated to exactly one base function Gⱼ (the one with the same index j).

Subsequently, a first example of set of base functions according to the invention will be derived. For this purpose, we only consider wavefront functions which do not have constant (piston) and one-degree (tilt)terms. Mathematically, such wavefront functions have zero gradient at origin: (equation 9) ${W}_{0} ⊂ \left\{W∈{C}^{2}\left(D\right); W\left(0, 0\right)=\frac{\partial W}{\partial x}\left(0, 0\right)=\frac{\partial W}{\partial y}\left(0, 0\right)=0\right\}$ and associated vergence functions: (equation 10) ${V}_{0} = \left\{\left(r, t\right)↦\frac{i}{r}\frac{\partial {W}_{p} \left(r, t\right)}{\partial r}; W∈{W}_{0}\right\}$

Every bivariate function V in V₀ is the vergence function of a unique wavefront W₀ , which is given by its polar expression: (equation 11) ${W}_{p} \left(r, t\right) = \frac{1}{2} {\int }_{0}^{r} ρ V \left(ρcos\left(t\right),ρsin\left(t\right)\right) d ρ$

Using polar coordinates, a wavefront in W₀ may be expanded (uniform convergence to Wₚ of a finite sum of more-than-one-valuation (no piston, no tilt) polynomials results from Stone-Weierstrass theorem) as a linear combination of "pure" normalized modes A^{m}ₙ, as follows (equation 12) ${W}_{p} = {\sum }_{n \geq 2} \left({\sum }_{m ∈ {I}_{n}} {a}_{n}^{m} \left(W\right) {A}_{n}^{m}\right) ≃ {W}_{p}^{{N}_{max}} = {\sum }_{n = 2}^{{N}_{max}} \left({\sum }_{m ∈ {I}_{n}} {a}_{n}^{m} \left(W\right) {A}_{n}^{m}\right)$

Where Nₘₐₓ (also just N) specifies the maximal chosen degree to express wavefront W (equation 13) ${A}_{n}^{m} = {R}_{\left(n\right)} ⊗ {T}_{\left(m\right)} : = \left(r, t\right) ↦ {R}_{\left(n\right)} \left(r\right) {T}_{\left(m\right)} \left(t\right)$ $\left\{\begin{matrix}{I}_{n} = \left\{-n,-n+2,…,+n-2,+n\right\} \\ {R}_{\left(n\right)} \left(r\right) = \sqrt{n + 1} {r}^{n} \\ {T}_{\left(m\right)} \left(t\right) = \left\{\begin{matrix}\sqrt{2} cos \left(mt\right) & if m > 0 \\ 1 & if m = 0 \\ \sqrt{2} sin \left(-mt\right) & if m < 0\end{matrix}\right.\end{matrix}\right.$

Both, R₍ₙ₎ and T₍ₘ₎ have been "normalized", i.e. (equation 14) ${\int }_{0}^{1} {\left({R}_{\left(n\right)}\left(r\right)\right)}^{2} 2 r d r = 1 and \frac{1}{2 π} {\int }_{- π}^{+ π} {\left({T}_{\left(m\right)}\left(t\right)\right)}^{2} d t = 1 ,$ so that (equation 15) $\frac{1}{π} {\int }_{0}^{1} \left({\int }_{- π}^{+ π} {\left({A}_{n}^{m}\left(r, t\right)\right)}^{2} d t\right) r d r = \frac{1}{π} {\int }_{0}^{1} \left({\int }_{- π}^{+ π} {\left({R}_{\left(n\right)}\left(r\right)\right)}^{2} {\left({T}_{\left(m\right)}\left(t\right)\right)}^{2} d t\right) r d r = 1 .$

The Zernike functions or also the newly derived base functions can be distinguished in low degree (LD) functions with n equal to 2 or small and high degree (HD) functions with n equal to 3 or larger.

For a wavefront W in W₀, LD functions shrink down to three modes (three base function) (equation 16) ${\sum }_{m ∈ {I}_{2}} {a}_{2}^{m} \left(W\right) {A}_{2}^{m} \left(r, t\right) = {r}^{2} \left({a}_{2}^{- 2} sin\left(2t\right)+{a}_{2}^{0}+{a}_{3}^{+ 2} cos\left(2t\right)\right)$ whereas the HD functions are: (equation 17) ${\sum }_{n \geq 3} \left({\sum }_{m ∈ {I}_{n}} {a}_{n}^{m} \left(W\right) {A}_{n}^{m}\right) ≃ {\sum }_{n = 3}^{{N}_{max}} \left({\sum }_{m ∈ {I}_{n}} {a}_{n}^{m} \left(W\right) {A}_{n}^{m}\right) .$

To compute a convenient complete basis, an inner product can be defined on the vergence space V₀ as follows (equation 18) $\left〈{V}^{\left(1\right)},V\left(2\right)\right〉 = \frac{1}{π} {\int }_{0}^{1} \left({\int }_{- π}^{+ π} {V}^{\left(1\right)} \left(r, t\right) {V}^{\left(2\right)} \left(r, t\right) d t\right) r d r .$

The family (equation 19) ${\left({R}_{\left(n\right)}⊗{T}_{\left(m\right)}\right)}_{n \geq 0 , m ∈ {I}_{n + 2}}$ is complete in Hilbertian space V₀ and a convenient basis for an euclidian space is needed (equation 20) ${V}_{0}^{{N}_{max}} = \left\{{\sum }_{n = 2}^{{N}_{max}} \left({\sum }_{m ∈ {I}_{n}} {a}_{n}^{m} \left(W\right) \frac{i}{r} \frac{\partial {A}_{n}^{m}}{\partial r}\right) ; W ∈ {W}_{0}\right\}$

An orthonormal decomposition basis (the set of base functions of the first embodiment) can be obtained through a Gramm-Schmidt orthogonalization process (applied on R₍ₙ₎). This results in the following formula for the orthonormal vergence modes or set of base functions of the first embodiment: (equation 21) ${{V}^{˜}}_{n}^{m} = {R}_{n - 2}^{{m}^{˜}} ⊗ {T}_{\left(m\right)}$ $where {m}^{˜} = \left\{\begin{matrix}max \left\{\left|m\right|-2,1\right\} & if m is odd \\ max \left\{\left|m\right|-2,0\right\} & if m is even\end{matrix}\right.$

If we choose N=6, which caps up vergence functions (or base functions) at fourth degree, the following 25 vergence modes or base functions are obtained: (equation 22) $\begin{matrix}\left\{\begin{matrix}{{G}^{˜}}_{1} = {{V}^{˜}}_{2}^{- 2} = {R}_{0} ⊗ {T}_{\left(-2\right)} : \left(r, t\right) ↦ \sqrt{2} sin \left(2t\right) \\ {{G}^{˜}}_{2} = {{V}^{˜}}_{2}^{0} = {R}_{0} ⊗ {T}_{0} : \left(r, t\right) ↦ 1 \\ {{G}^{˜}}_{3} = {{V}^{˜}}_{2}^{+ 2} = {R}_{\left(0\right)} ⊗ {T}_{\left(+2\right)} : \left(r, t\right) ↦ \sqrt{2} cos \left(2t\right)\end{matrix}\right. \\ \left\{\begin{matrix}{{G}^{˜}}_{4} = {{V}^{˜}}_{3}^{- 3} = {R}_{1}^{1} ⊗ {T}_{\left(-3\right)} : \left(r, t\right) ↦ 2 r sin \left(3t\right) \\ {{G}^{˜}}_{5} = {{V}^{˜}}_{3}^{- 1} = {R}_{1}^{1} ⊗ {T}_{\left(-1\right)} : \left(r, t\right) ↦ 2 r sin \left(t\right) \\ {{G}^{˜}}_{6} = {{V}^{˜}}_{3}^{+ 1} = {R}_{1}^{1} ⊗ {T}_{\left(+1\right)} : \left(r, t\right) ↦ 2 r cos \left(t\right) \\ {{G}^{˜}}_{7} = {{V}^{˜}}_{3}^{+ 3} = {R}_{1}^{1} ⊗ {T}_{\left(+3\right)} : \left(r, t\right) ↦ 2 r cos \left(3t\right)\end{matrix}\right. \\ \left\{\begin{matrix}{{G}^{˜}}_{8} = {{V}^{˜}}_{4}^{- 4} = {R}_{2}^{2} ⊗ {T}_{\left(-4\right)} : \left(r, t\right) ↦ \sqrt{6} {r}^{2} sin \left(4t\right) \\ {{G}^{˜}}_{9} = {{V}^{˜}}_{4}^{- 2} = {R}_{2}^{0} ⊗ {T}_{\left(-2\right)} : \left(r, t\right) ↦ \sqrt{6} \left(2{r}^{2}-1\right) sin \left(2t\right) \\ {{G}^{˜}}_{10} = {{V}^{˜}}_{4}^{0} = {R}_{2}^{0} ⊗ {T}_{\left(0\right)} : \left(r, t\right) ↦ \sqrt{3} \left(2{r}^{2}-1\right) \\ {{G}^{˜}}_{11} = {{V}^{˜}}_{4}^{+ 2} = {R}_{2}^{0} ⊗ {T}_{\left(+2\right)} : \left(r, t\right) ↦ \sqrt{6} \left(2{r}^{2}-1\right) cos \left(2t\right) \\ {{G}^{˜}}_{12} = {{V}^{˜}}_{4}^{+ 4} = {R}_{2}^{2} ⊗ {T}_{\left(+4\right)} : \left(r, t\right) ↦ \sqrt{6} {r}^{2} cos \left(4t\right)\end{matrix}\right.\end{matrix} \begin{matrix}\left\{\begin{matrix}{{G}^{˜}}_{13} = {{V}^{˜}}_{5}^{- 5} = {R}_{3}^{3} ⊗ {T}_{\left(-5\right)} : \left(r, t\right) ↦ 2 \sqrt{2} {r}^{3} sin \left(5t\right) \\ {{G}^{˜}}_{14} = {{V}^{˜}}_{5}^{- 3} = {R}_{3}^{1} ⊗ {T}_{\left(-3\right)} : \left(r, t\right) ↦ 2 \sqrt{2} \left(3{r}^{3}-2r\right) sin \left(3t\right) \\ {{G}^{˜}}_{15} = {{V}^{˜}}_{5}^{- 1} = {R}_{3}^{1} ⊗ {T}_{\left(-1\right)} : \left(r, t\right) ↦ 2 \sqrt{2} \left(3{r}^{3}-2r\right) sin \left(t\right) \\ {{G}^{˜}}_{16} = {{V}^{˜}}_{5}^{+ 1} = {R}_{3}^{1} ⊗ {T}_{\left(+1\right)} : \left(r, t\right) ↦ 2 \sqrt{2} \left(3{r}^{3}-2r\right) cos \left(t\right) \\ {{G}^{˜}}_{17} = {{V}^{˜}}_{5}^{3} = {R}_{3}^{1} ⊗ {T}_{\left(+3\right)} : \left(r, t\right) ↦ 2 \sqrt{2} \left(3{r}^{3}-2r\right) cos \left(3t\right) \\ {{G}^{˜}}_{18} = {{V}^{˜}}_{5}^{- 5} = {R}_{3}^{3} ⊗ {T}_{\left(+5\right)} : \left(r, t\right) ↦ 2 \sqrt{2} {r}^{3} cos \left(5t\right)\end{matrix}\right. \\ \left\{\begin{matrix}{{G}^{˜}}_{19} = {{V}^{˜}}_{6}^{- 6} = {R}_{4}^{4} ⊗ {T}_{\left(-6\right)} : \left(r, t\right) ↦ \sqrt{10} {r}^{4} sin \left(6t\right) \\ {{G}^{˜}}_{20} = {{V}^{˜}}_{6}^{- 4} = {R}_{4}^{2} ⊗ {T}_{\left(-4\right)} : \left(r, t\right) ↦ \sqrt{10} \left(4{r}^{4}-3{r}^{2}\right) sin \left(4t\right) \\ {{G}^{˜}}_{21} = {{V}^{˜}}_{6}^{- 2} = {R}_{4}^{0} ⊗ {T}_{\left(-2\right)} : \left(r, t\right) ↦ \sqrt{10} \left(6{r}^{4}-6{r}^{2}+1\right) sin \left(2t\right) \\ {{G}^{˜}}_{22} = {{V}^{˜}}_{6}^{0} = {R}_{4}^{0} ⊗ {T}_{\left(0\right)} : \left(r, t\right) ↦ \sqrt{5} \left(6{r}^{4}-6{r}^{2}+1\right) \\ {{G}^{˜}}_{23} = {{V}^{˜}}_{6}^{+ 2} = {R}_{4}^{0} ⊗ {T}_{\left(+2\right)} : \left(r, t\right) ↦ \sqrt{10} \left(6{r}^{4}-6{r}^{2}+1\right) cos \left(2t\right) \\ {{G}^{˜}}_{24} = {{V}^{˜}}_{6}^{+ 4} = {R}_{4}^{2} ⊗ {T}_{\left(+4\right)} : \left(r, t\right) ↦ \sqrt{10} \left(4{r}^{4}-3{r}^{2}\right) cos \left(4t\right) \\ {{G}^{˜}}_{25} = {{V}^{˜}}_{6}^{+ 4} = {R}_{4}^{4} ⊗ {T}_{\left(+6\right)} : \left(r, t\right) ↦ \sqrt{10} {r}^{4} cos \left(6t\right)\end{matrix}\right.\end{matrix}$

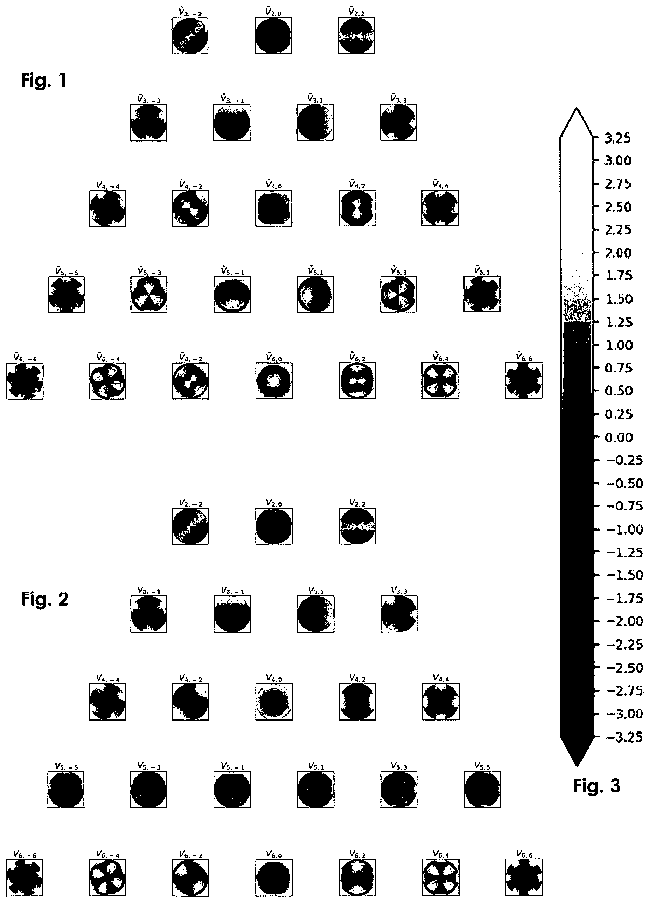

Fig. 1 shows the plots of the 25 base functions according to the first embodiment over r and t. This set of base functions is here shown until N=6 or J=25. The developed set of base functions can obviously also be used with higher or lower N or J. This set of base functions according to the first embodiment has the advantage that it describes vergence maps as well as curvature maps and other derivative wavefront maps. The orthogonality among all base functions allows a mathematically precise decomposition of any derivative wavefront map. This improves significantly the interpretability of the coefficients related to the base functions. In addition, each base function is normalized which also improves the interpretability of the coefficients significantly as different coefficients have different size only due to the different types of aberrations and not because the base functions are not normalized.

However, this set of base functions has also a disadvantage. The following HD base functions ${{V}^{˜}}_{4}^{- 2} , {{V}^{˜}}_{4}^{0} , {{V}^{˜}}_{4}^{+ 2} , {{V}^{˜}}_{6}^{- 2} , {{V}^{˜}}_{6}^{0} , {{V}^{˜}}_{6}^{+ 2}$ contain also LD components, i.e. components not depending on the radius r or in other words r⁰. For certain applications such as for ophthalmology, the LD coefficients describing defocus and astigmatism are the more important than the HD coefficients. For example, in ophthalmology, LD aberrations can still be corrected with spectacle lenses. A strong HD coefficient with n=4 or 6 with m=+2, 0 or -2 would thus generate also LD components which could falsify the LD coefficients. Therefore, a set of base functions according to a second embodiment could be better in certain cases. The idea is to remove all LD components in the HD components. This assures that only the LD coefficients fully describe aberrations regarding defocus and astigmatism without any falsification by HD coefficients. Thus, a clinician can interpret very easily the LD coefficients. For example, the LD coefficients for an eye lens expressed in dioptre can directly be read and used to prescribe a corresponding type of correction lens (defocus, astigmatism) with the corresponding correction strength (dioptre of coefficients). This might cause that the HD base functions which were adapted are not any more orthogonal to the LD base functions. However, all HD base functions remain orthogonal among each other so that this is acceptable considering the clinical advantage. The second set of base functions thus is not any more fully orthonormal decomposition basis, but does not any more mix LD and HD leading to a "crowned" truncated pyramid of base functions ${V}_{2}^{- 2} {V}_{2}^{0} {V}_{2}^{+ 2}$ ${V}_{3}^{- 3} {V}_{3}^{- 1} {V}_{3}^{+ 1} {V}_{3}^{+ 3}$ ${V}_{4}^{- 4} {V}_{4}^{- 2} {V}_{4}^{0} {V}_{4}^{+ 2} {V}_{4}^{+ 4}$ ${V}_{5}^{- 5} {V}_{5}^{- 3} {V}_{5}^{- 1} {V}_{5}^{+ 1} {V}_{5}^{+ 3} {V}_{5}^{+ 5}$ ${V}_{6}^{- 6} {V}_{6}^{- 4} {V}_{6}^{- 2} {V}_{6}^{0} {V}_{6}^{+ 2} {V}_{6}^{+ 4} {V}_{6}^{+ 6}$ where orthonormal LD vergence modes or base functions are (equation 23) $\left\{\begin{matrix}{V}_{2}^{- 2} = {R}_{\left(0\right)} ⊗ {T}_{\left(-2\right)} : \left(r, t\right) ↦ \sqrt{2} sin \left(2t\right) \\ {V}_{2}^{0} = {R}_{\left(0\right)} ⊗ {T}_{\left(0\right)} : \left(r, t\right) ↦ 1 \\ {V}_{2}^{+ 2} = {R}_{\left(0\right)} ⊗ {T}_{\left(+2\right)} : \left(r, t\right) ↦ \sqrt{2} cos \left(2t\right)\end{matrix}\right.$

And orthonormal HD vergence modes, obtained through Gramm-Schmidt orthonormalization process. (equation 24) ${V}_{n}^{m} = {R}_{n - 2}^{\hat{m}} ⊗ {T}_{\left(m\right)}$ where $\hat{m} = \left\{\begin{matrix}max \left\{\left|m\right|-2,1\right\} & if m is odd \\ max \left\{\left|m\right|-2,2\right\} & if m is even\end{matrix}\right.$ ${R}_{n}^{m} : = r ↦ \sqrt{n + 1} {\sum }_{0 \leq k \leq \frac{n - \left|m\right|}{2}} {\left(-1\right)}^{k} \frac{\left(n-k\right) !}{k ! \left(\frac{n - m}{2}-k\right) ! \left(\frac{n + m}{2}-k\right) !} {r}^{n - 2 k}$

If we choose N=6, which caps up vergence/base functions at fourth degree, get the following 22 HD vergence modes/base functions: (equation 25) $\begin{matrix}\left\{\begin{matrix}{G}_{4} = {V}_{3}^{- 3} = {R}_{1}^{1} ⊗ {T}_{\left(-3\right)} : \left(r, t\right) ↦ 2 r sin \left(3t\right) \\ {G}_{5} = {V}_{3}^{- 1} = {R}_{1}^{1} ⊗ {T}_{\left(-1\right)} : \left(r, t\right) ↦ 2 r sin \left(t\right) \\ {G}_{6} = {V}_{3}^{+ 1} = {R}_{1}^{1} ⊗ {T}_{\left(+1\right)} : \left(r, t\right) ↦ 2 r cos \left(t\right) \\ {G}_{7} = {V}_{3}^{+ 3} = {R}_{1}^{1} ⊗ {T}_{\left(+3\right)} : \left(r, t\right) ↦ 2 r cos \left(3t\right)\end{matrix}\right. \\ \left\{\begin{matrix}{G}_{8} = {V}_{4}^{- 4} = {R}_{2}^{2} ⊗ {T}_{\left(-4\right)} : \left(r, t\right) ↦ \sqrt{6} {r}^{2} sin \left(4t\right) \\ {G}_{9} = {V}_{4}^{- 2} = {R}_{2}^{2} ⊗ {T}_{\left(-2\right)} : \left(r, t\right) ↦ \sqrt{6} {r}^{2} sin \left(2t\right) \\ {G}_{10} = {V}_{4}^{0} = {R}_{2}^{2} ⊗ {T}_{\left(0\right)} : \left(r, t\right) ↦ \sqrt{3} {r}^{2} \\ {G}_{11} = {V}_{4}^{+ 2} = {R}_{2}^{2} ⊗ {T}_{\left(+2\right)} : \left(r, t\right) ↦ \sqrt{6} {r}^{2} cos \left(2t\right) \\ {G}_{12} = {V}_{4}^{+ 4} = {R}_{2}^{2} ⊗ {T}_{\left(+4\right)} : \left(r, t\right) ↦ \sqrt{6} {r}^{2} cos \left(4t\right)\end{matrix}\right.\end{matrix} \begin{matrix}\left\{\begin{matrix}{G}_{13} = {V}_{5}^{- 5} = {R}_{3}^{3} ⊗ {T}_{\left(-5\right)} : \left(r, t\right) ↦ 2 \sqrt{2} {r}^{3} sin \left(5t\right) \\ {G}_{14} = {V}_{5}^{- 3} = {R}_{3}^{1} ⊗ {T}_{\left(-3\right)} : \left(r, t\right) ↦ 2 \sqrt{2} \left(3{r}^{3}-2r\right) sin \left(3t\right) \\ {G}_{15} = {V}_{5}^{- 1} = {R}_{3}^{1} ⊗ {T}_{\left(-1\right)} : \left(r, t\right) ↦ 2 \sqrt{2} \left(3{r}^{3}-2r\right) sin \left(t\right) \\ {G}_{16} = {V}_{5}^{+ 1} = {R}_{3}^{1} ⊗ {T}_{\left(+1\right)} : \left(r, t\right) ↦ 2 \sqrt{2} \left(3{r}^{3}-2r\right) cos \left(t\right) \\ {G}_{17} = {V}_{5}^{3} = {R}_{3}^{1} ⊗ {T}_{\left(+3\right)} : \left(r, t\right) ↦ 2 \sqrt{2} \left(3{r}^{3}-2r\right) cos \left(3t\right) \\ {G}_{18} = {V}_{5}^{- 5} = {R}_{3}^{3} ⊗ {T}_{\left(+5\right)} : \left(r, t\right) ↦ 2 \sqrt{2} {r}^{3} cos \left(5t\right)\end{matrix}\right. \\ \left\{\begin{matrix}{G}_{19} = {V}_{6}^{- 6} = {R}_{4}^{4} ⊗ {T}_{\left(-6\right)} : \left(r, t\right) ↦ \sqrt{10} {r}^{4} sin \left(6t\right) \\ {G}_{20} = {V}_{6}^{- 4} = {R}_{4}^{2} ⊗ {T}_{\left(-4\right)} : \left(r, t\right) ↦ \sqrt{10} \left(4{r}^{4}-3{r}^{2}\right) sin \left(4t\right) \\ {G}_{21} = {V}_{6}^{- 2} = {R}_{4}^{2} ⊗ {T}_{\left(-2\right)} : \left(r, t\right) ↦ \sqrt{10} \left(4{r}^{4}-3{r}^{2}\right) sin \left(2t\right) \\ {G}_{22} = {V}_{6}^{0} = {R}_{4}^{2} ⊗ {T}_{\left(0\right)} : \left(r, t\right) ↦ \sqrt{5} \left(4{r}^{4}-3{r}^{2}\right) \\ {G}_{23} = {V}_{6}^{+ 2} = {R}_{4}^{2} ⊗ {T}_{\left(+2\right)} : \left(r, t\right) ↦ \sqrt{10} \left(4{r}^{4}-3{r}^{2}\right) cos \left(2t\right) \\ {G}_{24} = {V}_{6}^{+ 4} = {R}_{4}^{2} ⊗ {T}_{\left(+4\right)} : \left(r, t\right) ↦ \sqrt{10} \left(4{r}^{4}-3{r}^{2}\right) cos \left(4t\right) \\ {G}_{25} = {V}_{6}^{+ 6} = {R}_{4}^{4} ⊗ {T}_{\left(+6\right)} : \left(r, t\right) ↦ \sqrt{10} {r}^{4} cos \left(6t\right)\end{matrix}\right.\end{matrix}$

Fig. 2 shows the plots of the 25 base functions according to the second embodiment over r and t. The vergence LD-HD decomposition follows: (equation 26) $\left\{\begin{matrix}{V}_{L} = {v}_{2}^{- 2} \left(W\right) {V}_{2}^{- 2} + {v}_{2}^{0} \left(W\right) {V}_{2}^{0} + {v}_{2}^{+ 2} \left(W\right) {V}_{2}^{+ 2} \\ {V}_{H} = {\sum }_{n = 3}^{{N}_{max}} \left({\sum }_{m ∈ {I}_{n + 2}} {v}_{n}^{m} \left(W\right) {V}_{n}^{m}\right)\end{matrix}\right.$

V=V_{L}+V_{H} is the unique decomposition of any vergence function V₀^{N} as sum of a low-degree portion and a high-degree portion without mixing them. Since the HD-basis in this embodiment is orthonormal, computing vₙ^{m}(W) HD-coefficients in the above equation is straightforward as soon as the estimate V_{L} is calculated.

Subsequently, the example of an empirical vergence map will be described. The local wavefront slopes are measured by an aberrometer, e.g. by a lenslet array of a Shack-Hartmann aberrometer. This results for example in a spot pattern.

### Spot pattern (cartesian grid)

$C = \left(\begin{matrix}\left({x}_{1}, {y}_{1}\right) \\ \left({x}_{2}, {y}_{2}\right) \\ ⋮ \\ \left({x}_{N}, {y}_{N}\right)\end{matrix}\right) = \left(\begin{matrix}\left({r}_{1} cos\left({t}_{1}\right),{r}_{1} sin\left({t}_{1}\right)\right) \\ \left({r}_{2} cos\left({t}_{2}\right),{r}_{2} sin\left({t}_{2}\right)\right) \\ ⋮ \\ \left({r}_{N} cos\left({t}_{N}\right),{r}_{N} sin\left({t}_{N}\right)\right)\end{matrix}\right)$

### Spot pattern (polar grid)

$P = \left(\begin{matrix}\left({r}_{1}, {t}_{1}\right) \\ \left({r}_{2}, {t}_{2}\right) \\ ⋮ \\ \left({r}_{N}, {t}_{N}\right)\end{matrix}\right)$

### Empirical vergence map

${V}_{e} = \left(\begin{matrix}{v}_{1} \\ {v}_{2} \\ ⋮ \\ {v}_{N}\end{matrix}\right) = \left(\begin{matrix}{V}_{e} \left({r}_{1}, {t}_{1}\right) \\ {V}_{e} \left({r}_{2}, {t}_{2}\right) \\ ⋮ \\ {V}_{e} \left({r}_{N}, {t}_{N}\right)\end{matrix}\right)$

An "empirical" scalar product which corresponds to a discrete version of equation 18 (equation 27) $\left〈{V}_{e}^{\left(1\right)}, {V}_{e}^{\left(2\right)}\right〉 = \frac{1}{N} {\sum }_{j = 1}^{N} {V}_{e}^{\left(1\right)} \left({r}_{j}, {t}_{j}\right) {V}_{e}^{\left(2\right)} \left({r}_{j}, {t}_{j}\right)$

In order to separate LD from HD, instead of using a reconstruction of a "theoretical" vergence map V such that $\left(\begin{matrix}V \left({r}_{1}, {t}_{1}\right) \\ V \left({r}_{2}, {t}_{2}\right) \\ ⋮ \\ V \left({r}_{N}, {t}_{N}\right)\end{matrix}\right) ≃ \left(\begin{matrix}{v}_{1} \\ {v}_{2} \\ ⋮ \\ {v}_{N}\end{matrix}\right)$

It can be started with a direct reconstruction of V_{L} through its coordinates in an LD-basis using the local domination of V_{L} over V_{H} around the origin. The following three asymptotic properties (equation 28) ${lim}_{ρ \to 0} \frac{1}{{πρ}^{2}} {\int }_{0}^{ρ} \left({\int }_{- π}^{+ π} V \left(r, t\right) \sqrt{2} sin\left(2t\right)dt\right) r d r = {v}_{0}^{- 2} \left(W\right)$${lim}_{ρ \to 0} \frac{1}{{πρ}^{2}} {\int }_{0}^{ρ} \left({\int }_{- π}^{+ π} V \left(r, t\right)dt\right) r d r = {v}_{0}^{0} \left(W\right)$${lim}_{ρ \to 0} \frac{1}{{πρ}^{2}} {\int }_{0}^{ρ} \left({\int }_{- π}^{+ π} V \left(r, t\right) \sqrt{2} cos\left(2t\right)dt\right) r d r = {v}_{0}^{+ 2} \left(W\right)$ allow to estimate the three LD coefficients using an averaging method over a small radius disk Dᵣ around the centre/origin of the polar coordinate system. (equation 29) ${v}_{0}^{- 2} \left(W\right) ≃ \frac{1}{# \left\{j s.t. {r}_{j}\leq r\right\}} {\sum }_{js . t . {r}_{j} \leq r} {V}_{e} \left({r}_{j}, {t}_{j}\right) \sqrt{2} sin \left(2{t}_{j}\right)$ ${v}_{0}^{0} \left(W\right) ≃ \frac{1}{# \left\{j s.t. {r}_{j}\leq r\right\}} {\sum }_{js . t . {r}_{j} \leq r} {V}_{e} \left({r}_{j}, {t}_{j}\right)$ ${v}_{0}^{+ 2} \left(W\right) ≃ \frac{1}{# \left\{j s.t. {r}_{j}\leq r\right\}} {\sum }_{js . t . {r}_{j} \leq r} {V}_{e} \left({r}_{j}, {t}_{j}\right) \sqrt{2} cos \left(2{t}_{j}\right)$

In the example of the eye lens, the radius disk would correspond to a small central circle of the pupil around the centre point or optical axis of the pupil. Using these estimates, the HD coefficients vₙ^{m} of VH=V-VL can be determined with the orthonormal basis of the HD functions: (equation 30) ${v}_{n}^{m} \left(W\right) ≃ \frac{1}{N} {\sum }_{j = 1}^{N} {V}_{H} \left({r}_{j}, {t}_{j}\right) {V}_{n}^{m} \left({r}_{j}, {t}_{j}\right)$

Another example for a derivative wavefront map is a dioptric power map or curvature map. Defining the dioptric power as an inverse of the radius of curvature of the wavefront, it can be related to the second order radial partial derivative of the wavefront W: (equation 31) $D ≃ c \frac{{\partial }^{2} W}{\partial {r}^{2}}$

Where c is a constant which depends on the wavefront and radial distance units. Zernike expansion of a wavefront map provides a similar development for dioptric maps (choosing c=1) (equation 32) $D = \frac{{\partial }^{2} W \left(r, t\right)}{\partial {r}^{2}} = {\sum }_{n \geq 0} \left({\sum }_{m ∈ {I}_{n}} {z}_{n}^{m} \left(W\right) \frac{{\partial }^{2} {Z}_{n}^{m}}{\partial {r}^{2}}\right)$

However, choosing the second derivative of the Zernike polynomials/functions entails both a lack of orthonormality and a mixture of LD and HD modes. Therefore, it is better to proceed as above for the vergence maps using the same basis.

Thus, the two developed set of base functions with N=6 can be resumed as in the following table:

| D | O | j | n | m | $G {′}_{j} \left(r, t\right) = V {′}_{n}^{m} \left(r, t\right)$ | ${G}_{j} \left(r, t\right) = {V}_{n}^{m} \left(r, t\right)$ |
|---|---|---|---|---|---|---|
| LD | 0 | 1 | 2 | -2 | $\sqrt{2} sin \left(2t\right)$ | $\sqrt{2} sin \left(2t\right)$ |
| | | 2 | 2 | 0 | 1 | 1 |
| | | 3 | 2 | 2 | $\sqrt{2} cos \left(2t\right)$ | $\sqrt{2} cos \left(2t\right)$ cos(2*t*) |
| | | | | | | |
| HD | 1 | 4 | 3 | -3 | 2r sin(3*t*) | 2r sin(3*t*) |
| | | 5 | 3 | -1 | 2r sin(*t*) | 2r sin(*t*) |
| | | 6 | 3 | 1 | 2r cos(*t*) | 2r cos(*t*) |
| | | 7 | 3 | 3 | 2r cos(3*t*) | 2r cos(3*t*) |
| | | | | | | |
| | 2 | 8 | 4 | -4 | $\sqrt{6} {r}^{2} sin \left(4t\right)$ | $\sqrt{6} {r}^{2} sin \left(4t\right)$ |
| | | 9 | 4 | -2 | $\sqrt{6} \left(2{r}^{2}-1\right) sin \left(2t\right)$ | $\sqrt{6} {r}^{2} sin \left(2t\right)$ |
| | | 10 | 4 | 0 | $\sqrt{3} \left(2{r}^{2}-1\right)$ | $\sqrt{3} {r}^{2}$ |
| | | 11 | 4 | 2 | $\sqrt{6} \left(2{r}^{2}-1\right) cos \left(2t\right)$ | $\sqrt{6} {r}^{2} cos \left(2t\right)$ |
| | | 12 | 4 | 4 | $\sqrt{6} {r}^{2} cos \left(4t\right)$ | $\sqrt{6} {r}^{2} cos \left(4t\right)$ |
| | | | | | | |
| | 3 | 13 | 5 | -5 | $2 \sqrt{2} {r}^{3} sin \left(5t\right)$ | $2 \sqrt{2} {r}^{3} sin \left(5t\right)$ |
| | | 14 | 5 | -3 | $2 \sqrt{2} \left(3{r}^{3}-2r\right) sin \left(3t\right)$ | $2 \sqrt{2} \left(3{r}^{3}-2r\right) sin \left(3t\right)$ |
| | | 15 | 5 | -1 | $2 \sqrt{2} \left(3{r}^{3}-2r\right) sin \left(t\right)$ | $2 \sqrt{2} \left(3{r}^{3}-2r\right) sin \left(t\right)$ |
| | | 16 | 5 | 1 | $2 \sqrt{2} \left(3{r}^{3}-2r\right) cos \left(t\right)$ | $2 \sqrt{2} \left(3{r}^{3}-2r\right) cos \left(t\right)$ |
| | | 17 | 5 | 3 | $2 \sqrt{2} \left(3{r}^{3}-2r\right) cos \left(3t\right)$ | $2 \sqrt{2} \left(3{r}^{3}-2r\right) cos \left(3t\right)$ |
| | | 18 | 5 | 5 | $2 \sqrt{2} {r}^{3} cos \left(5t\right)$ | $2 \sqrt{2} {r}^{3} cos \left(5t\right)$ |
| | | | | | | |
| | 4 | 19 | 6 | -6 | $\sqrt{10} {r}^{4} sin \left(6t\right)$ | $\sqrt{10} {r}^{4} sin \left(6t\right)$ |
| | | 20 | 6 | -4 | $\sqrt{10} \left(4{r}^{4}-3{r}^{2}\right) sin \left(4t\right)$ | $\sqrt{10} \left(4{r}^{4}-3{r}^{2}\right) sin \left(4t\right)$ |
| | | 21 | 6 | -2 | $\sqrt{10} \left(6{r}^{4}-6{r}^{2}+1\right) sin \left(2t\right)$ | $\sqrt{10} \left(4{r}^{4}-3{r}^{2}\right) sin \left(2t\right)$ |
| | | 22 | 6 | 0 | $\sqrt{5} \left(6{r}^{4}-6{r}^{2}+1\right)$ | $\sqrt{5} \left(4{r}^{4}-3{r}^{2}\right)$ |
| | | 23 | 6 | 2 | $\sqrt{10} \left(6{r}^{4}-6{r}^{2}+1\right) cos \left(2t\right)$ | $\sqrt{10} \left(4{r}^{4}-3{r}^{2}\right) cos \left(2t\right)$ |
| | | 24 | 6 | 4 | $\sqrt{10} \left(4{r}^{4}-3{r}^{2}\right) cos \left(4t\right)$ | $\sqrt{10} \left(4{r}^{4}-3{r}^{2}\right) cos \left(4t\right)$ |
| | | 25 | 6 | 6 | $\sqrt{10} {r}^{4} cos \left(6t\right)$ | $\sqrt{10} {r}^{4} cos \left(6t\right)$ |

The first column of the table indicates the degree D of the function that is if the function of the corresponding row relates to a LD function or a HD function. The second column indicates the order O of the corresponding base function, i.e. the maximum power O of r in the base function. For example, order 2 base functions have at least one component which depend on r². The fourth and sixth columns indicate the indices n and m as used for developing the base functions above (similar to the indices n and m of the Zernike polynomials). As can be seen, the order O is related to the index n with O=n-2. The third column inidcates a simplified index j which indexes the set of base functions by subsequent natural numbers. The sixth column refers to the set of base functions according to the first embodiment with full orthonormality or orthogonality among all base functions. The seventh column refers to the set of base functions according to the second embodiment with full orthonormality or orthogonality among all HD base functions, but no orthogonality between LD and HD functions. In the sixth columns, the LD components in the HD base functions are highlighted in bold. An LD component of an HD base function Gⱼ is any summative component of order O=0, i.e. with a component aⱼr⁰=a being constant with respect to r. A summative component means any (non-zero) component which is added to the HD base function (and which does not depend on r). For example, the HD base functions of order O=2 with j=9, 10, 11 have such a LD component -sqrt(6)sin(2t), -sqrt(3) and -sqrt(6)cos(2t), respectively. Also, the HD base functions of order O=4 with j=21, 22, 23 have such a LD component - sqrt(10)sin(2t), -sqrt(5) and -sqrt(10)cos(2t), respectively. In the base functions of the second embodiment, these LD components have been removed as indicated in the bold base functions in the seventh column. The remaining base functions (without LD components) are the same in the first and second embodiment.

Even if the set of base functions according to the first and second embodiment are the most preferred ones, the set of base functions according to the invention could still be varied with the inventive properties. The set of base functions of both embodiments could be generalized as being directly proportional to the following set of polynomials:

| D | O | j | n | m | $G {′}_{j} \left(r, t\right) = V {′}_{n}^{m} \left(r, t\right) ∼$ | with |
|---|---|---|---|---|---|---|
| LD | 0 | 1 | 2 | -2 | a₁ sin(2t) | a₁ ≠ 0 |
| | | 2 | 2 | 0 | *a*₂ | *a*₂ ≠ 0 |
| | | 3 | 2 | 2 | *a*₃ cos(2*t*) | *a*₃ ≠ 0 |
| | | | | | | |
| HD | 1 | 4 | 3 | -3 | *b*₄*r* sin(3*t*) | *b*₄ ≠ 0 |
| | | 5 | 3 | -1 | *b*₅*r* sin(*t*) | *b*₅ ≠ 0 |
| | | 6 | 3 | 1 | *b*₆*r* cos(*t*) | *b*₆ ≠ 0 |
| | | 7 | 3 | 3 | *b*₇*r* cos(3*t*) | *b*₇ ≠ 0 |
| | | | | | | |
| | 2 | 8 | 4 | -4 | *c*₈*r*² sin(4*t*) | *c*₈ ≠ 0 |
| | | 9 | 4 | -2 | (*c*₉*r*² + *a*₉) sin(2*t*) | *c*₉ ≠ 0 ${a}_{9} ∈ ℝ$ |
| | | 10 | 4 | 0 | (*c*₁₀*r*² + *a*₁₀) | *c*₁₀ ≠ 0 ${a}_{10} ∈ ℝ$ |
| | | 11 | 4 | 2 | (*c*₁₁*r*² + *a*₁₁) cos(2*t*) | *c*₁₁ ≠ 0, ${a}_{11} ∈ ℝ$ |
| | | 12 | 4 | 4 | *c*₁₂*r*² cos(4*t*) | *c*₁₂ ≠ 0 |
| | | | | | | |
| | 3 | 13 | 5 | -5 | *d*₁₃*r*³ sin(5*t*) | *d*₁₃ ≠ 0 |
| | | 14 | 5 | -3 | (*d*₁₄*r*³ - *b*₁₄*r*) sin(3*t*) | *d*₁₄ ≠ 0, *b*₁₄ ≠ 0 |
| | | 15 | 5 | -1 | (*d*₁₅*r*³ - *b*₁₅*r*) sin(*t*) | *d*₁₅ ≠ 0, *b*₁₅ ≠ 0 |
| | | 16 | 5 | 1 | (*d*₁₆*r*³ - *b*₁₆*r*) cos(*t*) | *d*₁₆ ≠ 0, *b*₁₆ ≠ 0 |
| | | 17 | 5 | 3 | (*d*₁₇*r*³ - *b*₁₇*r*) cos(3*t*) | *d*₁₇ ≠ 0, *b*₁₇ ≠ 0 |
| | | 18 | 5 | 5 | *d*₁₈*r*³ cos(5*t*) | *d*₁₈ ≠ 0 |
| | | | | | | |
| | 4 | 19 | 6 | -6 | *e*₁₉*r*⁴ sin(6*t*) | *e*₁₉ ≠ 0 |
| | | 20 | 6 | -4 | (*e*₂₀*r*⁴ + *c*₂₀*r*²) sin(4*t*) | *e*₂₀, *c*₂₀ ≠ 0 |
| | | 21 | 6 | -2 | (*e*₂₁*r*⁴ + *c*₂₁*r*² + *a*₂₁) sin(2*t*) | *e*₂₁, *c*₂₁ ≠ 0, ${a}_{21} ∈ ℝ$ |
| | | 22 | 6 | 0 | (*e*₂₂*r*⁴ + *c*₂₂*r*² + *a*₂₂) | *e*₂₂, *c*₂₂ ≠ 0, ${a}_{22} ∈ ℝ$ |
| | | 23 | 6 | 2 | (*e*₂₃*r*⁴ + *c*₂₃*r*² + *a*₂₃) cos(2*t*) | *e*₂₃, *c*₂₃ ≠ 0, *a* ${a}_{23} ∈ ℝ$ |
| | | 24 | 6 | 4 | (*e*₂₄*r*⁴ + *c*₂₄*r*²) cos(4*t*) | *e*₂₄, *c*₂₄ ≠ 0 |
| | | 25 | 6 | 6 | *e*₂₅*r*⁴ cos(6*t*) | *e*₂₅ ≠ 0 |

The coefficients aj, bj, cj, dj, ej will subsequently be called multiplicators aj, bj, cj, dj, ej to distinguish them from the coefficients gj weighting the base functions Gj.

The base functions are preferably normalized. This makes the coefficients gj associated to the base functions Gj comparable among each other. A base function is normalized, if a normalization function over each base function results in the same value, preferably one, and/or follows a common concept. The normalization function of a base function contains preferably an integral of a function of the base function over the radius r and over the angle t. The normalization function is for example equation 15. A normalized base function shall include also base functions which are not normalized, but whose computed coefficients are corrected/normalized by the multiplicator necessary to normalize the base function. An alternative normalization could be to normalize the base functions to peak-to-valley. Thus, the function is scaled so that the difference between its minimum and maximum is equal to 1. This is no longer a normalization in the analytical sense but rather an interpretative one. In this normalization, the value of each coefficient gj can be directly interpreted by the ophthalmologist as the amplitude of the dioptric variation associated with the specified aberration. For instance, for coma (which varies linearly with r), a coefficient of 3 D would indicate that the aberration induces a linear gradient of 3 D across the analyzed zone. For spherical aberration (varying with r²), a coefficient of 4 D would mean there is a 4 D difference between the center and the edge of the analyzed area, and so on.

The base functions are constituted by a set of LD base functions and a set of HD base functions.

The base functions of LD or of order O=0 or with n=2 or with j=1, ..., 3 are constant with respect to the radius r. The base function with m=0 or j=2 is constant also with respect to the angle t. The base functions with m=-2 or 2 or j=1 or 2 are a function of sin(2t) or cos(2t). The multiplicator a1, a2 and/or a3 are/is unequal to zero. a1, a2, a3 can be any (positive or negative) value of the real number space except zero. a1, a2 and/or a3 are preferably positive values. a1 and a3 are preferably the same value. However, they could also assume different values. Preferably, the ratio between a2 and a1 or a3 is one to square root of 2. Preferably, the LD base functions are orthogonal to each other. Preferably, the LD base functions are normalized. Preferably, the LD base functions are orthonormal to each other.

The set of HD base functions comprise at least one order O=1, preferably at least two orders O=1,2 or more. In one embodiment, the set of HD base functions comprise preferably base functions of orders one to three (O=1, ..., 3). In one embodiment, the set of HD base functions comprise preferably base functions of orders one to four (O=1, ..., 4). Other embodiments with higher orders O=1, ..., N with N being 5 or higher are obviously possible.

The HD base functions are preferably orthogonal to each other. This means that each HD base function is orthogonal to each other distinct HD base function. In the first embodiment, the HD base functions are actually also orthogonal to the LD base functions. Thus, all base functions of the first embodiment are orthogonal among each other. In the second embodiment, some HD base functions (e.g., j=9, 10, 11, 21, 22, 23) are not orthogonal to the LD base functions. This orthogonality at least in HD allows to compute the coefficients gⱼ in equation 8 very easily as shown for example in equation 30.

The HD base functions of order O=1 or with n=3 or with j=4, ..., 7 are linear with respect to the radius r, i.e. depend on r. The HD base functions of order O=1 are a function of sin(3t), sin(t), cos(t) or cos(3t). The multiplicator b4, b5, b6 and/or b7 are/is unequal to zero. The multiplicator b4, b5, b6 and/or b7 can be any (positive or negative) value of the real number space (except zero). The multiplicator b4, b5, b6 and/or b7 are preferably positive values. Two, three or all 4 of the multiplicators b4, b5, b6 and/or b7 have preferably the same value, preferably two. However, they could also assume different values. Preferably, the HD base functions of order O=1 are orthogonal to each other. Preferably, the HD base functions of order O=1 are normalized. Preferably, the HD base functions of order =1 are orthonormal to each other. Preferably, the ratio between the multiplicator b4, b5, b6 and/or b7 and the multiplicator a1 or a3 is square root of two. Preferably, the ratio between the multiplicator b4, b5, b6 and/or b7 and the multiplicator a2 is two.

The HD base functions of order O=2 or with n=4 or with j=8, ..., 12 are parabolic with respect to the radius r, i.e. depend on the radius power 2 or depend on a polynomial of second order in radius r. Some or four of the HD base functions of order O=2 (m=-4, -2, 2, 4 or j=8, 9, 11, 12) are a functions of sin(4t), sin(2t), cos(2t) or cos(4t). The HD base function of order =2 and m=0 or j=10 is preferably constant in the angle t. The multiplicator c8, c9, c10, c11 and/or c12 are/is unequal to zero. The multiplicator c8, c9, c10, c11 and/or c12 can be any (positive or negative) value of the real number space (except zero). The multiplicator c8, c9, c10, c11 and/or c12 is/are preferably positive values. Two of the multiplicators c8, c9, c10, c11 and c12 have preferably the same value, preferably c8=c12 and/or c11=c9. However, they could also assume different values. Preferably, one or more of the multiplicators c8, c9, c11 and c12 are preferably a multiple (including one) of $\sqrt{6} like {c}_{j} =$ $n \sqrt{6}$ *with n being a positive or negative non* - *zero integer* and j = 8, 9, 11, 12. Preferably, the multiplicator c10 is preferably a multiple of $\sqrt{3} like {c}_{10} =$ $n \sqrt{3}$ *with n being a positive or negative non* - *zero integer.* In the first embodiment, for the multiplicators one or more of the following conditions hold: ${c}_{8} = \sqrt{6} , {c}_{9} = 2 \sqrt{6} ,$ ${c}_{10} = 2 \sqrt{3} , {c}_{11} = 2 \sqrt{6} , {c}_{12} = \sqrt{6}$. In the second embodiment, for the multiplicators one or more of the following conditions hold: ${c}_{8} = \sqrt{6} , {c}_{9} = \sqrt{6} , {c}_{10} = \sqrt{3} , {c}_{11} = \sqrt{6} , {c}_{12} =$ $\sqrt{6}$. The multiplicator a9, a10 and/or a11 of the LD components can be unequal to zero (in case of the first embodiment) or could be zero (in case of the second embodiment). The following information about the multiplicators a9, a10 and/or a11 refers obviously only to the case when they are unequal to zero. The multiplicators a9, a10 and/or a11 can be any (positive or negative) value of the real number space except zero. Preferably, a9, a10 and/or a11 are negative values and/or are of opposed polarity/sign with respect to the multiplicator c8, c9, c10, c11 and/or c12. The multiplicator a9 and a11 have preferably the same value. However, they could also assume different values. The ratio between cj and aj of the same index j is 2. Preferably, the HD base functions of order O=2 are orthogonal to each other. Preferably, the HD base functions of order O=2 are normalized. Preferably, the HD base functions of order =2 are orthonormal to each other.

The HD base functions of order O=3 or with n=5 or with j=13, ..., 18 are cubic with respect to the radius r, i.e. depend on the radius power 3 or depend on a polynomial of third order in radius r. The HD base functions of order O=3 (m=-5, -3, -1, 1, 3, 5 or j=13, ... 18) are a function of sin(5t), sin(3t), sin(t), cos(t), cos(3t) or cos(5t). The multiplicator dj with j=13, ..., 18 is unequal to zero. The multiplicator dj with j=13, ..., 18 can be any (positive or negative, preferably positive) value of the real number space (except zero). Two, three, four, five or six of the multiplicators dj with j=13, ..., 18 have preferably the same value. However, they could also assume different values. The multiplicator bj with j=14, ..., 17 is unequal to zero. The multiplicator bj with j=14, ..., 17 can be any (positive or negative, preferably negative) value of the real number space except zero. Preferably, the multiplicators bj with j=14, ..., 17 are negative values and/or are of opposed polarity/sign with respect to the multiplicator dj of the same index j. multiplicator bj with j=14, ..., 17 have preferably the same value. However, they could also assume different values. The ratio between dj and bj of the same index j is 3 to 2 (3 divided by 2). Preferably, the HD base functions of order O=3 are orthogonal to each other. Preferably, the HD base functions of order O=3 are normalized. Preferably, the HD base functions of order O=3 are orthonormal to each other.

The HD base functions of order O=4 or with n=6 or with j=19, ..., 25 depend on the radius power 4 or depend on a polynomial of fourth order in radius r. The HD base functions of order O=4 (m=-6, -2, -2, 2, 4, 6 or j=19, ... 25) are a function of sin(6t), sin(4t), sin(2t), cos(2t), cos(4t) or cos(6t). One function (m=0 or j=22) of order O=4 is constant with respect to the angle t. Preferably, one or more or all of the multiplicators ej for j=19, 20, 21, 23, 24, 25 being a multiple (including one) of $\sqrt{10}$ with ${e}_{j} = n \sqrt{10}$ and n being a positive or negative non-zero integer. Preferably, one or more or all of the multiplicators cj for j=20, 21, 23, 24 being a multiple (including one) of $\sqrt{10}$ with ${c}_{j} = n \sqrt{10}$ and n being a positive or negative non-zero integer. The integer n can be different for different indexes. Preferably, one or more or all of the multiplicators aj for j= 21, 23 being a multiple (including one) of $\sqrt{10}$ with ${a}_{j} = n \sqrt{10}$ and n being a positive or negative integer (being for the second embodiment zero). The integer n can be different for different indexes. Preferably, one or more of the multiplicators e22, c22, a22 are a multiple (including one) of $\sqrt{5}$ with ${e}_{j} = n 1 \sqrt{5} , {c}_{j} =$ $n 2 \sqrt{5} {c}_{22} = n 3 \sqrt{5}$ and n1, n2, n3 being a positive or negative integer (for ej and cj a non-zero integer and for aj including zero). The multiplicator ej with j=19, ..., 25 is unequal to zero. The multiplicator ej with j=19, ..., 25 can be any (positive or negative, preferably positive) value of the real number space (except zero). Two of the multiplicator ej with j=19, ..., 25 have preferably the same value, preferably in the first embodiment, e21=e23=6sqrt(10)), e20=e24=4sqrt(10)) and/or e19=e25(=sqrt(10)) and in the second embodiment, e21=e23=4sqrt(10)), e20=e24=4sqrt(10)) and/or e19=e25(=sqrt(10)). However, they could also assume different values. The multiplicator cj with j=20, ..., 24 is unequal to zero. The multiplicator cj with j=20, ..., 24 can be any (positive or negative, preferably negative) value of the real number space except zero. Preferably, the multiplicator cj with j=20, ..., 24 are negative values and/or are of opposed polarity/sign with respect to the multiplicator dj of the same index j. Two of the multiplicators cj with j=20, ..., 24 have preferably the same value, preferably in the first embodiment, c21=c23(=-6sqrt(10)) and/or c20=c24(=-3sqrt(10)), and in the scond embodiment, c21=c23(=-3sqrt(10)) and/or c20=c24(=-3sqrt(10)). However, they could also assume different values. The multiplicator aj for j=21, 22, 23 refer to the LD components in those functions j=21, 22, 23 and can be unequal to zero (in case of the first embodiment) or could be zero (in case of the second embodiment). The following information about the multiplicators aj for j=21, 22, 23 refer obviously only to the case when they are unequal to zero. The multiplicators aj for j=21, 22, 23 can be any (positive or negative, preferably positive) value of the real number space except zero. Preferably, aj for j=21, 22, 23 are positive values and/or are of opposed polarity/sign with respect to the multiplicator cj of the same index j and/or are of the same polarity/sign with respect to the multiplicator ej of the same index j. The multiplicator aj for j=21, 22, 23 have preferably the same value, preferably one. However, they could also assume different values. The ratio between cj and aj of the same index j is -6 (-6 divided by 1). The ratio between ej and aj of the same index j is 6 (6 divided by 1). Preferably, the HD base functions of order O=4 are orthogonal to each other. Preferably, the HD base functions of order O=4 are normalized. Preferably, the HD base functions of order O=4 are orthonormal to each other.

For simplicity, the indices j of the multiplicators aj, bj, cj, dj and ej have sometimes been put as a subscript (e.g. aⱼ) and sometimes as normal character (e.g. aj) without any difference in meaning.

Each base function represents a certain aberration of the form, especially for lens objects. The subsequent table summarizes which base function describes which kind of aberration:

| **D** | **O** | **j** | **n** | **m** | **Optical meaning** |
|---|---|---|---|---|---|
| LD | 0 | 1 | 2 | -2 | Oblique astigmatism |
| | | 2 | 2 | 0 | Defocus (longitudinal position) |
| | | 3 | 2 | 2 | Vertical astigmatism |
| HD | 1 | 4 | 3 | -3 | Vertical trefoil |
| | | 5 | 3 | -1 | Vertical coma |
| | | 6 | 3 | 1 | Horizontal coma |
| | | 7 | 3 | 3 | Oblique trefoil |
| | 2 | 8 | 4 | -4 | Oblique quadrafoil |
| | | 9 | 4 | -2 | Oblique secondary astigmatism |
| | | 10 | 4 | 0 | Primary spherical |
| | | 11 | 4 | 2 | Vertical secondary astigmatism |
| | | 12 | 4 | 4 | Vertical quadrafoil |

The low degree base functions represent thus defocus (j=2) and oblique and vertical astigmatism (j=1 and j=3). In the field of ophthalmic optic, the LD base functions represent thus the most basic aberrations which can still be corrected by spectacle lenses. The HD base functions refer to more complex aberrations.

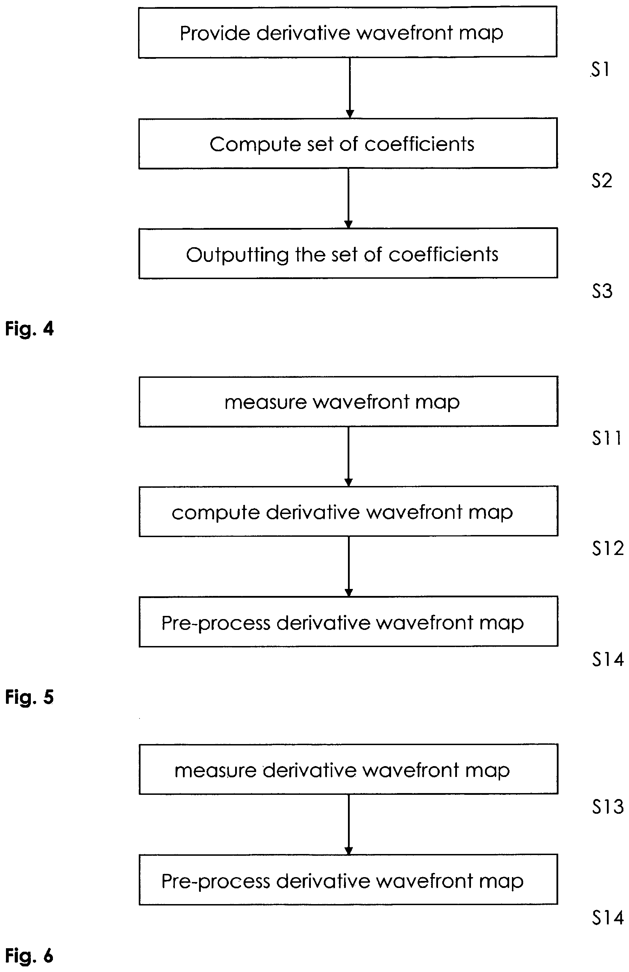

Fig. 4 shows an embodiment for a method to determine a shape/form of the object, preferably the lens object.

In step S1, a derivative wavefront map of the object is provided.

In step S2, a set of coefficients for the provided derivative wavefront map of S1 is computed based on a set of base functions according to the invention as explained above.

In step S3, the set of coefficients computed in step S2 are output.

Subsequently, the step S1 is described in more detail.

The step S1 of providing the derivative wavefront map can be realized in many ways. The derivative wavefront map can be received directly, e.g. from a measurement instrument or from a communication interface. The derivative map can also be computed from a received, measured or simulated wavefront map or received, measured or simulated (different) derivative wavefront map.

Fig. 5 shows a first embodiment of step S1 in which a wavefront map of the object is measured with a measurement instrument 1 in step S11. The derivative wavefront map is computed in step S12 based on the measured wavefront map. The derivative wavefront map can be calculated based on a derivative (of first or second order) of the wavefront map measured with respect to the radius r. The derivative wavefront map computed in step S12 could be for example a vergence map (see equation 4) or a curvature map (see equation 5). The step S1 could comprise further pre-processing step of the derivative wavefront map in step S14. The pre-processing step S14 could be performed equally on the wavefront map or on the derivative wavefront map. Such a pre-processing step could be the scaling and/or cutting of the derivative wavefront map to the same scale and form of the base functions, preferably a unity circle. Such a pre-processing step could be the transformation of the wavefront map in polar coordinates.

Fig. 6 shows a second embodiment of step S1 in which a derivative wavefront map is directly measured in step S13, e.g. a vergence map or a curvature map. The measured derivative wavefront map of step S13 can be optionally pre-processed in step S14 as explained above.

The embodiment of Fig. 5 and Fig. 6 could also be mixed by measuring a first derivative wavefront map in step S13 and to compute in step S12 a second derivative wavefront map based on the first derivative wavefront map (e.g. a curvature map from a vergence map or vice versa).

The wavefront map or the derivative wavefront map of steps S11 or S13 could be obtained also by simulation, e.g. ray tracing simulation, calculation or by other means instead of by measurement.

The step S1 could also be simply receiving the derivative wavefront map or the wavefront map.

Subsequently, the step S2 is described in more detail. Step S2 aims to compute the set of coefficients gⱼ (short gj) describing the derivative wavefront map provided in step S2.

In a preferred embodiment, the coefficients are expressed in dioptre. This allows clinicians to use the value of the coefficients (especially when normalized and/or orthogonalized) to select or prescribe a compensation lens (spectacle lens, contact lens, IOL or other) using directly the value of the coefficient.

In case of an orthogonal set of base functions G'j(r, t) as in the first embodiment, the coefficients gj(V) of the polynomial expansion series (equation 33) $V \left(r, t\right) \approx {\sum }_{j = 1}^{J} {g}_{j} \left(V\right) G {′}_{j} \left(r, t\right)$ of the derivative wavefront map V could be computed by the scalar product (equation 34) ${g}_{j} \left(V\right) = \left〈V\left(r, t\right),G{′}_{j}\left(r, t\right)\right〉$ of the derivative wavefront map V(r, t) with the j-th base function G'j(r, t) as defined in equation 18 or as defined in equation 27 for a discrete map. This results in a full set of coefficients gj(V) for j=1, ..., J describing the derivative wavefront map V.

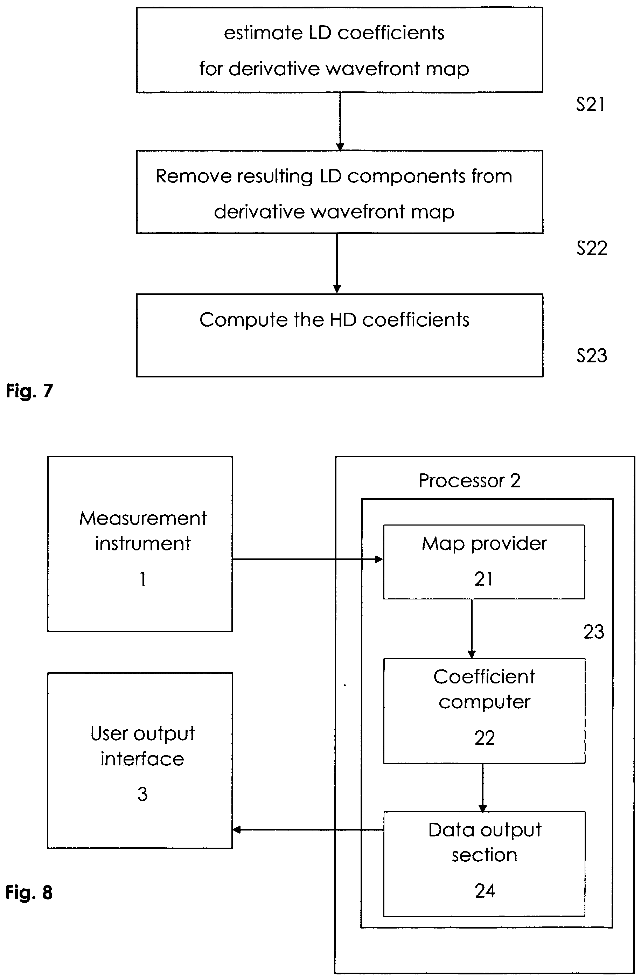

In case of a set of base functions which is orthogonal in the HD, but the HD base functions are not orthogonal to the LD base functions, the method for computing the coefficients from Fig. 7 could be used.

In step S21, LD coefficients gj(V) for j=1, ..., 3 are estimated. Preferably, the LD coefficients are obtained independent from the HD coefficients. This can be realized by fitting the LD polynomial expansion series (equation 35) ${\sum }_{j = 1}^{3} {g}_{j} \left(V\right) {G}_{j} \left(r, t\right)$ with base functions Gj(r, t) and its coefficients gj(V) for j=1, ..., 3 to the derivative wavefront map V(r, t) to obtain the LD coefficients gj(V) for j=1, ..., 3. However, other methods like a least square estimate, a simulation or other kind of optimization method could be applied to obtain the three LD coefficients.

In step S22, the estimated LD components of equation 35 are removed from the derivative wavefront map V(r,t), preferably by a subtraction, to obtain a residual HD derivative wavefront map V_{HD}(r,t) (equation 36) ${V}_{HD} \left(r, t\right) = V \left(r, t\right) - {\sum }_{j = 1}^{3} {g}_{j} \left(V\right) {G}_{j} \left(r, t\right) .$

In step S23, the HD coefficients gⱼ (V) for j=4, ..., J are computed based on the residual HD derivative wavefront map V_{HD}(r,t). Due to the orthogonality of the HD base functions Gj(r, t) with j=4, ..., J, the coefficients gj(V) of the polynomial HD expansion series (equation 37) ${V}_{HD} \left(r, t\right) \approx {\sum }_{j = 4}^{J} {g}_{j} \left(V\right) {G}_{j} \left(r, t\right)$ of the residual HD derivative wavefront map V could be computed by the scalar product (equation 38) ${g}_{j} \left(V\right) = \left〈{V}_{HD}\left(r, t\right),{G}_{j}\left(r, t\right)\right〉$ of the residual HD derivative wavefront map V_{HD}(r, t) with the j-th base function Gj(r, t) as defined in equation 18 or as defined in equation 27 for a discrete or empirical map. This results in a full set of HD coefficients gj(V) for j=4, ..., J describing the residual HD derivative wavefront map V.

Subsequently, the step S3 shall be explained in more detail. The step S3 could be realized by outputting the coefficients computed in S2 on a display. For example, the values of the coefficients could be represented by their corresponding alphanumerical values. Additionally or alternatively, each computed coefficient gj(V) could be illustrated based on the resulting j-th map obtained by the j-th base function Gj(r,t) multiplied by the j-th coefficient gj(V). Those maps are preferably illustrated as in Fig. 1 or 2 (just that each base map/function is multiplied with its computed coefficient gj(V)) in the pyramidal way (with truncated head) wherein functions of the same order O n are presented in the same row (or column in case of rotating the pyramid) and functions of the different orders O n are presented in different rows (or column in case of rotating the pyramid). Obviously, the user output interface 3 could simply be a communication interface which outputs the coefficients computed in step S2, e.g. to a server or to a connected computer. The coefficients can obviously also output via any other user output interface 3 than a communication interface or a display, e.g. by a audio output.

Fig. 8 schematically shows an embodiment of a system or device according to the invention. The system or device comprises at least a processor 2. Optionally, the system or device can comprise further a measurement instrument 1 and/or a user output interface 3.

The processor 2 receives a derivative wavefront map or a wavefront map (short received map) and computes based on the received map the coefficients as explained in step S2 above and outputs them as explained in step S3.

The processor 2 comprises a map provider 21 configured to provide the derivative wavefront map to the coefficient computer 22. The map provider 21 receives preferably a map of describing (the shape of the) object, preferably a wavefront map or a derivative wavefront map of the object. This wavefront map is preferably received from the measurement instrument 1, but could also be received from a data input means like a USB stick, an internet connection, etc. The map provider 21 is configured to perform one or more of the steps S12, S13 and/or S14. The map provider 21 can also just forward a received derivative wavefront map, if there are no pre-processing steps (S14) or computing steps (S12) to perform on the received map. The map provider 21 is preferably configured to compute the derivative wavefront map from the received wavefront map or received (different) derivative wavefront map as explained in detail in step S12. The map provider 21 is preferably configured to pre-process the map received and/or the computed derivative wavefront map as explained in more detail in step S14.

The processor 2 comprises a coefficient computer 22 for computing the coefficients of the derivative wavefront map computed and/or pre-processed in the map provider 21 or received at the processor 2. The coefficient computer 22 performs preferably the step S2 as explained above.

The processor comprises a data output section 24 configured to output coefficient data. The coefficient data can be simply the digital data containing any information based on the coefficients computed in step S2. The coefficient data can be the values of the coefficients themselves or image data to be displayed on a user output interface 3 representing the computed coefficients as explained above in step S3.

The processor 2 runs preferably a software 23 comprising the functionalities described above, especially the functionalities of the map provider 21 or step S12, S13 and/or S14, of the coefficient computer 22 or the step S2, and of the data output section 24 or the step S3. The software 23 can be stored in a memory associated or connected to the processor 2. The processor 2 can be realized in one or multiple computers. The computer can be a personal computer with the software 23 installed. The computer can also be a server or a mix between a server and a client computer connected to the server. However, it would also be possible to realize the functionalities of the processor 2 not by a software running on a CPU or other general processing unit, but by a specific processor like a microchip specific to the described functionalities of the processor 2.

The measurement instrument 1 is preferably an instrument configured to measure the wavefront map or derivative wavefront map of a curved object, preferably of a lens object, preferably of an eye lens. The measurement instrument 1 is preferably an aberrometer or an other instrument as already explained above. The measurement instrument 1 is preferably configured to perform the step S11 and/or S12. If the measurement instrument 1 is part of the device or system according to the invention, the measurement instrument 1 and the processor 2 can be realized in the same housing or in two (or more) distinct housings. If the processor 2 is not realized fully in the measurement instrument 1, the processor 2 could be realized in a distinct (personal) computer having a second housing, connected via a cable or other (wired or wireless) communication connection to the measurement instrument 1. If the processor 2 is realized fully on a remote server or cloud service, the measurement instrument 1 could be simply connected to the server or to the internet and the server 2 would realize all the functionalities of the processor 2 (without the user actually requiring a distinct housing for the processor 2). The processor 2 can also be distributed over two or more of the measurement instrument 1, a distinct housing for the processor and a remote server. For example, the pre-processing S14 and/or the computation of the derivative wavefront map S12 (functionalities of the map provider 21) can be realized in the processor part 2 of the measurement instrument 1 and the computation of the coefficients S2 or coefficient computer 22 can be realized in a distinct connected computer or server.

The user output interface 3 is preferably a display. However, as explained above, the user output interface 3 can also be a different means for outputting the coefficients computed in the coefficient computer 22. The user output interface 3 can be in the same housing of the measurement instrument 1 and/or the processor 2. For example, if the processor 2 is a distinct (personal) computer, the user output interface 3 is preferably realized in this computer. If the processor 2 is fully integrated in the measurement instrument 1, the user output interface 3 is preferably realized in the measurement instrument 1 as well. When the processor 2 is realized on a server, the user output interface 3 can be realized basically in any client computer connected to the same user account of the server which receives the map for computing the coefficients.

The main benefits of the invention will be explained for vergence in the field of ophthalmic optics.

### Direct and Efficient Construction

Radial vergence maps are derived directly from wavefront slope measurements (e.g., using a Shack-Hartmann sensor) or Zernike coefficients, relying on the radial derivative of the wavefront (*∂W*/*∂r*). This approach bypasses the need for wavefront phase reconstruction or computationally intensive techniques like ray tracing, resulting in a faster, simpler process that avoids assumptions about the wavefront error function. This efficiency enhances their practicality for both clinical and research applications.

### Intuitive Dioptric Representation

These maps express ocular aberrations in diopters across the pupil, resembling corneal power maps familiar to clinicians. For instance, defocus appears as a constant power value, astigmatism varies meridionally, and higher-order aberrations like coma manifest as linear power tilts. This format is more intuitive than traditional wavefront maps (in microns or wavelengths), making it easier for eye care professionals to interpret and relate to refractive corrections, thus bridging the gap between objective data and clinical practice.

### Strong Alignment with Subjective Refraction

Radial vergence maps excel at predicting subjective sphero-cylindrical refraction, achieving high correlation with patient-reported visual outcomes (e.g., R2 values exceeding 0.97 for spherical components). By translating wavefront data into power vectors they provide prescriptions that closely match perceived focus, outperforming methods based solely on Zernike coefficients or curvature-based power. This alignment ensures that corrections derived from these maps are clinically relevant and effective.

### Systematic and Quantitative Analysis

Using orthogonal base functions (at least for HD), radial vergence maps decompose aberrations into fundamental components-such as defocus, astigmatism, coma, and spherical aberration-each represented by a single term or coefficient. This systematic breakdown, calibrated in diopters, allows for precise, quantitative analysis without the complexity of infinite Zernike series, offering clear insights into the eye's optical properties and facilitating tailored interventions.

### Robustness Across Aberration Orders

These maps effectively handle both low- and higher-order aberrations, remaining robust even in the presence of complex wavefront errors (e.g., in normal eyes, post-LASIK, or mild keratoconus cases). Additionally, they can support phase reconstruction if needed, adding flexibility to their use.

## Claims

1. Method comprising the following steps:
providing a derivative wavefront map of an object;
computing a set of coefficients representing the derivative wavefront map, wherein each coefficient (gj) is related to a base function (Gj) of a set of base functions so that the linear combination of the set of base functions (Gj) each weighted by the coefficient (gj) of its base function (Gj) represents the derivative wavefront map (V), and
outputting the set of coefficients,
wherein the set of base functions comprises base functions *Gⱼ*(*r*, *t*) with j=1, ..., J depending on the radius r and the angle t, wherein the set of base functions comprise low degree base functions and high degree base functions, wherein the low degree base functions comprise ${G}_{1} \left(r, t\right) = {a}_{1} sin \left(2t\right) with {a}_{1} \neq 0 ,$ ${G}_{2} \left(r, t\right) = {a}_{2} with {a}_{2} \neq 0$ *and* ${G}_{3} \left(r, t\right) = {a}_{3} cos \left(2t\right) with {a}_{3} \neq 0 ;$ wherein the high degree base functions comprise ${G}_{4} \left(r, t\right) = {b}_{4} r sin \left(3t\right) with {b}_{4} \neq 0 ,$ ${G}_{5} \left(r, t\right) = {b}_{5} r sin \left(t\right) with {b}_{5} \neq 0 ,$ ${G}_{6} \left(r, t\right) = {b}_{6} r cos \left(t\right) with {b}_{6} \neq 0$
and ${G}_{7} \left(r, t\right) = {b}_{7} r cos \left(3t\right) with {b}_{7} \neq 0 ;$
wherein the high degree base functions are orthogonal to each other and/or wherein the set of base functions are normalized.

2. Method according to claim 1, wherein the high degree base functions are orthogonal to each other.

3. Method according to claim 1 or 2, wherein at least some of the high degree base functions are not orthogonal to the low degree base functions.

4. Method according to one of claims 1 to 3, wherein the high degree base functions have no components independent of the radius r or have no components of order zero with respect to the radius.

5. Method according to one of claims 1 to 3, wherein the step of computing the coefficients comprises the steps of:
estimating the coefficients of the low degree base functions for the derivative map as low degree coefficients;
computing the low degree derivative wavefront map based on the low degree coefficients and the low degree base functions;
determining a high degree derivative wavefront map based on the derivative wavefront map and the low degree derivative wavefront map; and
computing the coefficients of the high degree base functions for the high degree derivative wavefront map as high degree coefficients. The low degree coefficients and the high degree coefficients provide thus the set of coefficients for the set of base functions.

6. Method according to claim 5, wherein the low degree coefficients are estimated by fitting the linear combination of the three low degree base functions weighted by the three low degree coefficients such to the derivative wavefront maps such to the derivative wavefront map that a central portion/zone of the derivative wavefront map is considered more than an external portion/zone of the derivative wavefront map for the fitting.

7. Method according to claim 1 or 2, wherein the set of base functions constitute an orthogonal base, or each high degree base function is orthogonal to each low degree base function.

8. Method according to one of the claims 1 to 7, wherein the set of base functions are normalized.

9. Method according to one of the claims 1 to 8, wherein the coefficients are output in dioptre.

10. Method according to one of the claims 1 to 9, wherein the object is an ophthalmic lens, preferably an eye lens.

11. Method according to one of claims 1 to 10, wherein the multiplicators ${a}_{1} = {a}_{3} = \sqrt{2}$ and a₂ = 1 and the multiplicators *bⱼ =* 2 for j= 4, ..., 7 .

12. Method according to one of the claims 1 to 11, wherein the high degree base functions comprise the base functions ${G}_{8} \left(r, t\right) = {c}_{8} {r}^{2} sin \left(4t\right) with {c}_{8} \neq 0$ ${G}_{9} \left(r, t\right) = \left({c}_{9}{r}^{2}+{a}_{9}\right) sin \left(2t\right) with {c}_{9} \neq 0 , {a}_{9} ∈ ℝ$ ${G}_{10} \left(r, t\right) = \left({c}_{10}{r}^{2}+{a}_{10}\right) with {c}_{10} \neq 0 , {a}_{10} ∈ ℝ$ ${G}_{11} \left(r, t\right) = \left({c}_{11}{r}^{2}+{a}_{11}\right) cos \left(2t\right) with {c}_{11} \neq 0 , {a}_{11} ∈ ℝ$ ${G}_{12} \left(r, t\right) = {c}_{12} {r}^{2} cos \left(4t\right) with {c}_{12} \neq 0 .$

13. Method according to claim 12, wherein the multiplicators are a*ⱼ* = 0 for j= 9, ..., 11 .

14. Device comprising :
a map provider (21) for providing a derivative wavefront map of an object;
a coefficient computer (22) for computing a set of coefficients representing the derivative wavefront map, wherein each coefficient (gj) is related to a base function (Gj) of a set of base functions so that the linear combination of the set of base functions (Gj) each weighted by the coefficient (gj) of its base function (Gj) represents the derivative wavefront map (V), and
a data output section (24) for outputting the set of coefficients,
wherein the set of base functions comprise base functions *Gⱼ*(*r*, *t*) with j=1, ..., J depending on the radius r and the angle t, wherein the set of base functions comprise low degree base functions and high degree base functions, wherein the low degree base functions comprise ${G}_{1} \left(r, t\right) = {a}_{1} sin \left(2t\right) with {a}_{1} \neq 0 ,$ ${G}_{2} \left(r, t\right) = {a}_{2} with {a}_{2} \neq 0$ *and* ${G}_{3} \left(r, t\right) = {a}_{3} cos \left(2t\right) with {a}_{3} \neq 0 ;$ wherein the high degree base functions comprise ${G}_{4} \left(r, t\right) = {b}_{4} r sin \left(3t\right) with {b}_{4} \neq 0 ,$ ${G}_{5} \left(r, t\right) = {b}_{5} r sin \left(t\right) with {b}_{5} \neq 0 ,$ ${G}_{6} \left(r, t\right) = {b}_{6} r cos \left(t\right) with {b}_{6} \neq 0$
and ${G}_{7} \left(r, t\right) = {b}_{7} r cos \left(3t\right) with {b}_{7} \neq 0 ;$
wherein the high degree base functions are orthogonal to each other and/or wherein the set of base functions are normalized.

15. Device according to claim 14 comprising a measurement instrument (1), preferably an aberrometer, configured to measure a measurement map of the object, wherein the map provider (21) is configured to provide the derivative wavefront map of the object based on the measurement map from the measurement instrument (1).
